(19) Europäisches Patentamt / European Patent Office / Office européen des brevets

(11) **EP 4 353 265 A1**

(12) **EUROPEAN PATENT APPLICATION**
published in accordance with Art. 153(4) EPC

(43) Date of publication:
**17.04.2024 Bulletin 2024/16**

(21) Application number: **22820311.3**

(22) Date of filing: **09.06.2022**

(51) International Patent Classification (IPC):
*A61K 45/06* (2006.01)   *A61K 31/282* (2006.01)
*A61K 31/4745* (2006.01)   *A61K 31/513* (2006.01)
*A61K 31/519* (2006.01)   *A61K 39/395* (2006.01)
*A61P 1/04* (2006.01)   *A61P 1/18* (2006.01)
*A61P 35/00* (2006.01)   *A61P 35/04* (2006.01)
*A61P 37/02* (2006.01)   *A61P 43/00* (2006.01)

(52) Cooperative Patent Classification (CPC):
**A61K 31/282; A61K 31/4745; A61K 31/513;
A61K 31/519; A61K 39/395; A61K 45/06;
A61P 1/04; A61P 1/18; A61P 35/00; A61P 35/04;
A61P 37/02; A61P 43/00**

(86) International application number:
**PCT/JP2022/023300**

(87) International publication number:
**WO 2022/260132 (15.12.2022 Gazette 2022/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.06.2021 JP 2021097024
10.06.2021 JP 2021097027**

(71) Applicant: **ONO Pharmaceutical Co., Ltd.
Osaka-shi, Osaka 541-8526 (JP)**

(72) Inventor: **OKAMOTO, Tatsuya
Osaka-shi, Osaka 541-8564 (JP)**

(74) Representative: **Grünecker Patent- und
Rechtsanwälte
PartG mbB
Leopoldstraße 4
80802 München (DE)**

(54) **METHOD FOR TREATING CANCER THROUGH COMBINATION OF CD47 INHIBITOR, IMMUNE CHECKPOINT INHIBITOR, AND STANDARD THERAPY**

(57)    The present invention addresses the problem of providing an effective cancer treatment method. Provided is a cancer treatment method in which are combined a CD47 inhibitor, an immune checkpoint inhibitor (e.g., an anti-PD-1 antibody, etc.), and a standard therapy (e.g., combination therapy of bevacizumab or cetuximab added to FOLFOX therapy for unresectable advanced or recurrent colorectal cancer, FOLFIRINOX therapy or a low-dose regimen thereof for pancreatic cancer with distal metastases). The treatment method according to the present invention is useful in cancer treatment.

FIG. 1

**Description**

TECHNICAL FIELD

The present disclosure relates to a cancer treatment method (which may be abbreviated as the treatment method of the present invention) by combined use of a CD47 inhibitory substance, an immune checkpoint inhibitory substance, and a standard therapy.

BACKGROUND ART

CD47 is a 5-pass transmembrane type glycoprotein. CD47 expressed in cancer cells binds to signal regulatory protein alpha (SIRPα) expressed in macrophages and dendritic cells, thereby transmitting a "don't eat me" signal to macrophages, thus being involved in suppressing phagocytosis of cancer cells by macrophages. A CD47 inhibitory substance binds to CD47 on the cancer cell membrane, thereby inhibiting the "don't eat me" signal from being transmitted to macrophages, and promoting phagocytosis of cancer cells by macrophages, and therefore it is considered to be useful for cancer treatment (see Non-Patent Literature 1 to 4).

Patent Literature 1 discloses that a CD47 inhibitory substance promotes phagocytosis of cancer cells by macrophages and is useful as a cancer therapeutic agent (see Patent Literature 1).

On the other hand, various immune checkpoint molecules that interfere with the immune response against cancer exist in cancer cells and the microenvironment of cancer. An immune checkpoint inhibitory substance is a new therapeutic method for releasing an immunosuppressive mechanism and activating an immune reaction against cancer. Immune checkpoint inhibitory substances such as Ipilimumab, which is an anti-CTLA-4 (cytotoxic T lymphocyte-associated antigen-4) antibody, Nivolumab, Pembrolizumab, and the like, which are anti-PD-1 (programmed cell death-1) antibodies, have already obtained approval in Japan and other countries and are used in cancer therapy as an immune checkpoint inhibitory substance.

Pharmacotherapy is applied to radically unresectable advanced or recurrent colorectal cancer, and as a standard therapy, a combination therapy is available in which Bevacizumab, which is a molecularly targeted therapeutic agent for vascular endothelial growth factor (VEGF), or Cetuximab or Panitumumab, which is a molecularly targeted therapeutic agent for epidermal growth factor receptor (EGFR), is added to FOLFOX therapy using Fluorouracil (5-FU), Levofolinate, and Oxaliplatin in combination.

The treatment for unresectable pancreatic cancer having distant metastasis is mainly pharmacotherapy, and examples of the standard therapy include: FOLFIRINOX therapy (hereinafter, it may be abbreviated as "FFX therapy") in which a regimen containing Fluorouracil (5-FU) is used in combination with Oxaliplatin and Irinotecan; and modified FOLFIRINOX therapy (hereinafter, it may be abbreviated as "mFFX therapy") in which rapid administration of Fluorouracil is omitted and Irinotecan is reduced in expectation of toxicity reduction in FFX therapy.

Although a certain effect is observed in these therapeutic methods, there is an unmet need for treatment that further prolongs the survival period.

CITATIONS LISTS

Patent Literature

Patent Literature 1: WO2009/091601

Non Patent Literatures

Non Patent Literature 1: Trends in Cell Biology, Vol. 11 (3), pp. 130-135, 2001
Non Patent Literature 2: Journal of Experimental Medicine, Vol. 194 (4), pp. 541-549, 2001
Non Patent Literature 3: Journal of Immunology, vol. 174 (4), pp. 2004-201 1, 2005.
Non Patent Literature 4: Cell, Vol. 138 (2), pp. 286-299, 2009

SUMMARY OF INVENTION

TECHNICAL PROBLEMS

It is an object of the present invention to provide a new treatment method for cancer (e.g. colon cancer, pancreatic cancer).

SOLUTIONS TO PROBLEMS

As a result of intensive studies to achieve the above-described object, the present inventors have found that the use of a CD47 inhibitory substance and an immune checkpoint inhibitory substance in combination with standard therapy can be an effective cancer treatment method.

Accordingly, in an aspect, the followings are provided:

[1] An agent for suppressing progression of, suppressing recurrence of, and/or treating a solid cancer, comprising a CD47 inhibitory substance as an active ingredient, wherein the agent is administered in combination with a standard therapy and an immune checkpoint inhibitory substance; and
[2] an agent for suppressing progression of, suppressing recurrence of, and/or treating a solid cancer, comprising an immune checkpoint inhibitory substance as an active ingredient, wherein the agent is administered in combination with a standard therapy and a CD47 inhibitory substance.

ADVANTAGEOUS EFFECTS OF INVENTION

The treatment method of the present invention is useful for cancer treatment.

BRIEF DESCRIPTION OF DRAWINGS

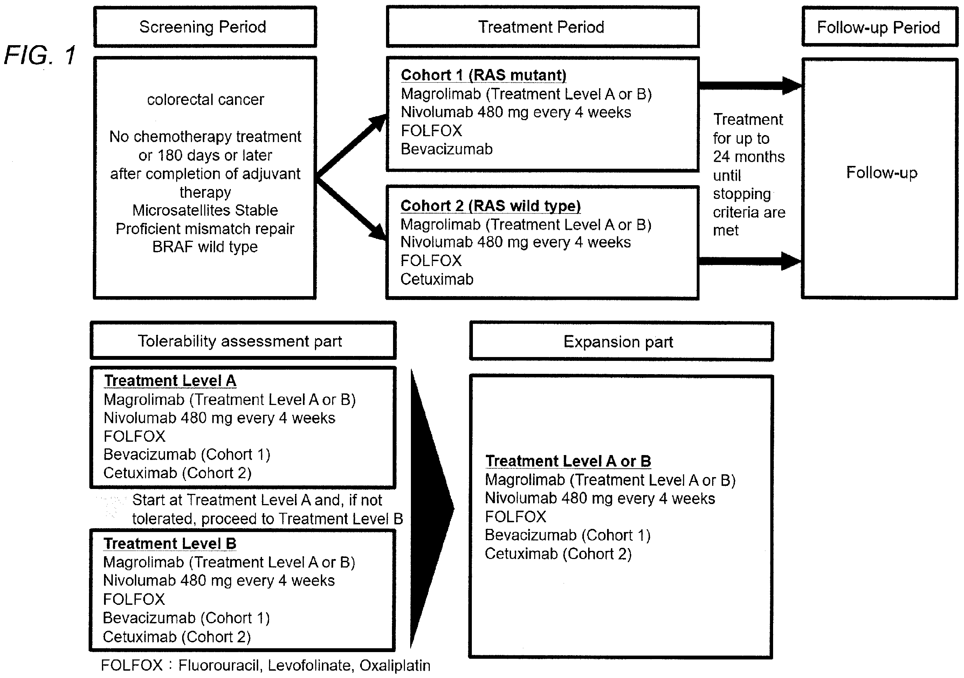

Fig. 1 shows an outline of a multi-center, open-label, uncontrolled study for evaluating tolerability, safety, and efficacy when combination in which Magrolimab or Nivolumab described below is used in combination with a combination therapy in which Bevacizumab or Cetuximab is added to FOLFOX therapy was applied to patients with radically unresectable advanced or recurrent colorectal cancer.

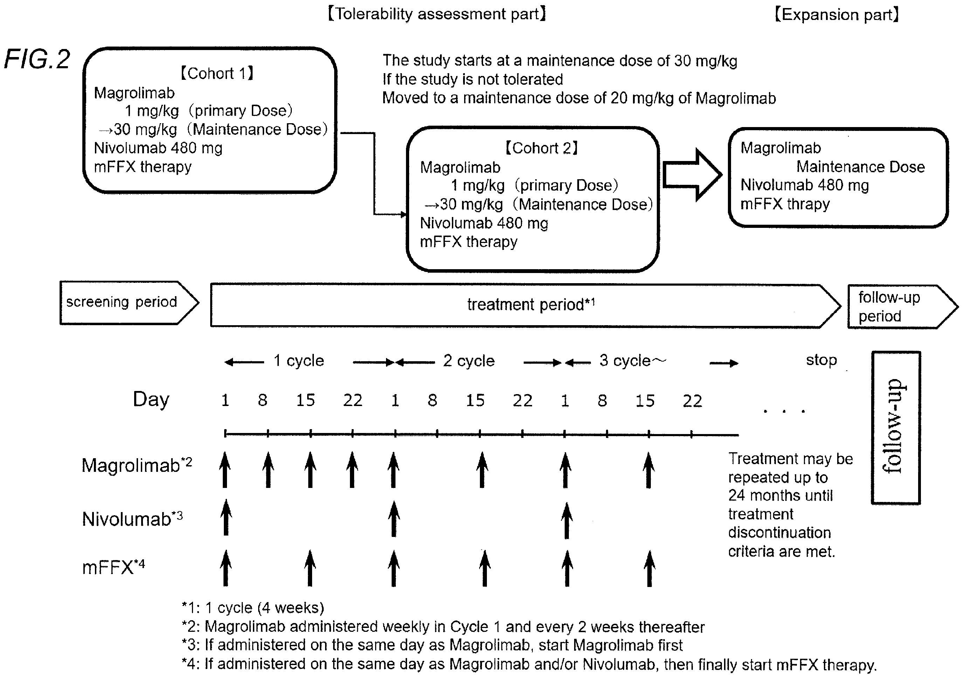

Fig. 2 shows an outline of a multi-center, open-label, uncontrolled study for evaluating tolerability, safety, and efficacy when combination in which Magrolimab or Nivolumab described below is used in combination with mFFX therapy was applied to patients having distant metastasis.

DESCRIPTION OF EMBODIMENTS

(1) CD47 inhibitory substance

In the present invention, the CD47 inhibitory substance is not particularly limited as long as it is a compound having a CD47 inhibitory action. In one embodiment, however, it is an agent (preferably an antibody, more preferably a monoclonal antibody) that inhibits the binding between CD47 and SIRP$\alpha$. Examples of the same include an anti-CD47 antibody and an anti-SIRP$\alpha$ antibody. The CD47 inhibitory substance may be an antigen-binding fragment (e.g. Fv, Fab, Fab', (Fab')$_2$, scFv, scFv-Fc) of the antibody. Examples of anti-CD47 antibody include Magrolimab (Hu5F9-G4), CC-90002, STI-6643, ZL-1201, TAY-018, SGN-CD47M, GenSci-059, Lemzoparlimab, Letaplimab, IMC-002, SHR-1603, AO-176, AVI-105, MIL-95, AK-117, HLX-24, SG-404, SY-102, IMM-01, KD-015, BAT-6004, ALX-148, SRF-231, TJ-011133, Letaplimab, TQB-2928, AL-008, JMT-601, and DSP-107. Examples of the anti-SIRP$\alpha$ antibody include ES-004, CTX-5861, ADU-1805, BI-765063, BYON-4228, FSI-189, and CC-95251. One kind of the CD47 inhibitory substance may be used singly, or two or more kinds of the same may be used in combination. The CD47 inhibitory substance is preferably an anti-CD47 antibody, and the anti-CD47 antibody is preferably Magrolimab (CAS No. 2169232-81-7). An antibody including heavy and light chain complementarity determining regions (CDRs) or variable regions (VR) of the above-described known antibody, or an antigen-binding fragment of the antibody, is also one aspect of the CD47 inhibitory substance. A further aspect of the anti-CD47 antibody is, for example, an antibody including heavy and light chain complementarity determining regions (CDRs) or variable regions (VR) of Magrolimab, or an antigen-binding fragment of the antibody. One aspect of the anti-CD47 antibody is, for example, an antibody that competes with Magrolimab for binding to CD47, an antibody that binds to the same CD47 epitope as that to which Magrolimab binds to, or an antigen-binding fragment of the antibody.

In the present invention, the CD47 inhibitory substance can be produced according to a known method, and for example, Magrolimab can be produced according to the method described in WO2011/143624.

The dose of the CD47 inhibitory substance used in the treatment method of the present invention varies depending on age, body weight, symptom, therapeutic effect, administration method, treatment time, and the like, but is adjusted so as to provide an optimum desired effect.

For example, the therapeutically effective dose (therapeutic dose) of the anti-CD47 antibody (for example, Magrolimab), in one embodiment, is from about 15 mg/kg to about 60 mg/kg (preferably from about 15 mg/kg to about 45 mg/kg, more preferably from about 15 mg/kg to about 30 mg/kg, and in a preferred embodiment, about 15 mg/kg, about 20 mg/kg, about 30 mg/kg, or about 45 mg/kg). In addition, the administration interval is, for example, half a week (3 days or 4 days), 1 week, or 2 weeks, and the single administration period is, for example, about 60 minutes or more, about 90 minutes or more, about 120 minutes or more, or about 2 hours ($\pm$ 30 minutes). Of course, as described above, since the dose varies depending on various conditions, an amount smaller than the above dose may be sufficient, or administration beyond the range may be necessary. In one embodiment, therapeutic doses may also be gradually increased to optimize safety and efficacy.

In one embodiment, a priming agent is administered before the therapeutically effective dose of the anti-CD47 antibody (for example, Magrolimab) is administered. A suitable priming agent contains an initial dose of an erythropoiesis-stimulating agent (ESA), and/or an anti-CD47 antibody (for example, Magrolimab). Following administration of the priming agent, a therapeutic dose of an anti-CD47 antibody (for example, Magrolimab) is administered after providing an effective period of time for increase of reticulocyte production (for example, at least about 3 days after administration of the priming agent (for example, after at least about 4 days, after at least about 5 days, after at least about 6 days, after at least about 7 days, after at least about 8 days, after at least about 9 days, or after at least about 10 days)). Regarding a particular suitable initial dose, etc., of the anti-CD47 antibody (for example, Magrolimab) in one embodiment, about 0.5 mg/kg to about 5 mg/kg (preferably about 1 mg/kg) is administered intravenously over a period of about 2.5 hours to about 6 hours (for example, from about 3 hours to about 4 hours, or about 3 hours ($\pm$ 30 minutes)). In one embodiment, about 1 mg/kg of an anti-CD47 antibody (for example, Magrolimab) is administered intravenously for the first administration, and about 15 mg/kg to about 30 mg/kg (preferably about 20 mg/kg or about 30 mg/kg) of the same in a single dose is administered intravenously at intervals of 1 week or 2 weeks for the second and subsequent administrations.

(2) Immune checkpoint inhibitory substance

In the present invention, the immune checkpoint molecule means a molecule that exerts an immunosuppressive function by transmitting a suppressive co-signal. As the immune checkpoint molecule, CTLA-4, PD-1, PD-L1 (programmed cell death-ligand 1), PD-L2 (programmed cell death-ligand 2), LAG-3 (lymphocyte activation gene 3), TIM3 (T cell immunoglobulin and mucin-3), BTLA (B and T lymphocyte attenuator), B7H3, B7H4, CD160, CD39, CD73, A2aR (adenosine A2a receptor), KIR (killer inhibitory receptor), VISTA (V-domain Ig-containing suppressor of T cell activation), IDOI (Indoleamine 2,3-dioxygenase), Arginase I, TIGIT (T cell immunoglobulin and ITIM domain), CD115, and the like are known (see Nature Reviews Cancer, 12, pp. 252-264, 2012; Cancer Cell, 27, pp. 450-461, 2015), but the immune checkpoint molecule is not particularly limited as long as it is a molecule having a function conforming to the definition.

In the present invention, the immune checkpoint inhibitory substance is a substance that inhibits the function of an immune checkpoint molecule. The immune checkpoint inhibitory substance is not particularly limited as long as it is a substance capable of suppressing the function (signal) of an immune checkpoint molecule. One kind of the immune checkpoint inhibitory substance may be used singly, or two or more kinds of the same may be used in combination. The immune checkpoint inhibitory substance is preferably an antibody (preferably a monoclonal antibody) or an antigen-binding fragment thereof (e.g. Fv, Fab, Fab', (Fab')$_2$, scFv, scFv-Fc).

Examples of the immune checkpoint inhibitory substance include an anti-PD-1 antibody (for example, Nivolumab, Cemiplimab (REGN-2810), Pembrolizumab (MK-3475), Spartalizumab (PDR-001), Tislelizumab (BGB-A317), AMP-514 (MEDI0680), Dostarlimab (ANB011/TSR-042), Toripalimab (JS001), Camrelizumab (SHR-1210), Genolimzumab (CBT-501), Sintilimab (IBI308), STI-A1110, ENUM 388D4, ENUM 244C8, GLS010, Retifanlimab (MGA012), Balstilimab (AGEN2034), CS1003, Serplulimab (HLX10), BAT-1306, AK105, AK103, BI 754091, LZM009, CMAB819, Sym021, Geptanolimab (GB226), SSI-361, JY034, HX008, ISU106, Budigalimab (ABBV181), Prolgolimab (BCD-100), Sasanlimab (PF-06801591), CX-188, Cetrelimab (JNJ-63723283), and Zimberelimab (AB122)), an anti-PD-L1 antibody (for example, Atezolizumab (RG7446/MPDL3280A), Avelumab (PF-06834635/MSB0010718C), Durvalumab (MEDI4736), BMS-936559, STI-1014, Envafolimab (KN035), Lodapolimab (LY3300054), HLX 20, SHR-1316, CS1001 (WBP3155), MSB2311, BGB-A333, KL-A167, CK-301, AK106, AK104, ZKAB001, FAZ053, CBT-502 (TQB2450), JS003, and CX-072), or an anti-CTLA-4 antibody (for example, Ipilimumab (MDX-010), Zalifrelimab (AGEN1884), and Tremelimumab), an anti-PD-L2 antibody, a PD-L1 fusion protein, a PD-L2 fusion protein (for example, AMP-224), an anti-Tim-3 antibody (for example, MBG453), an anti-LAG-3 antibody (for example, BMS-986016, LAG525), and an anti-KIR antibody (for example, Lirilumab). An antibody including heavy and light chain complementarity determining regions (CDRs) or variable regions (VR) of the above-described known antibody, or an antigen-binding fragment of the antibody, is also one aspect of the immune checkpoint inhibitory substance. A further aspect of the anti-PD-1 antibody is, for example, an antibody including heavy and light chain complementarity determining regions (CDRs) or variable regions (VR) of Nivolumab, or an antigen-binding fragment of the antibody. One aspect of the anti-PD-1 antibody is, for example, an antibody that competes with Nivolumab for binding to PD-1, an antibody that binds to the same PD-1 as that to which Nivolumab binds to, or an antigen-binding fragment of the antibody.

In the present invention, the immune checkpoint inhibitory substance is preferably an anti-PD-1 antibody, an anti-PD-L1 antibody, or an anti-CTLA-4 antibody, and more preferably an anti-PD-1 antibody or an anti-PD-L1 antibody. The anti-PD-1 antibody is preferably at least one selected from the group consisting of Nivolumab, Cemiplimab, Pembrolizumab, Spartalizumab, Tislelizumab, Toripalimab, Sintilimab, and Camrelizumab, the anti-PD-L1 antibody is preferably at least one selected from the group consisting of Atezolizumab, Avelumab, Durvalumab, and BMS -936559, and the anti-CTLA-4 antibody is preferably at least one selected from the group consisting of Ipilimumab and Tremelimumab. Furthermore, the anti-PD-1 antibody is more preferably at least one selected from the group consisting of Nivolumab, Cemiplimab, and Pembrolizumab, and it is still more preferably Nivolumab. In the present invention, the immune checkpoint inhibitory substance is preferably an anti-PD-1 antibody, and more preferably Nivolumab.

In the present invention, the immune checkpoint inhibitory substance can be produced by a known method. For example, Nivolumab can be produced according to the method described in WO2006/121168, Pembrolizumab can be produced according to the method described in WO2008/156712, BMS-936559 can be produced according to the method described in WO2007/005874, and Ipilimumab can be produced according to the method described in WO2001/014424.

In the present invention, any one or any two or more of these immune checkpoint inhibitory substances can be used in combination with the compound used in the present invention.

The dose of the immune checkpoint inhibitory substance used in the treatment method of the present invention varies depending on age, body weight, symptom, therapeutic effect, administration method, treatment time, and the like, but is adjusted so as to provide an optimum desired effect.

Regarding as an active ingredient of an immune checkpoint inhibitory substance, for example, about 1 mg/kg (body weight) to about 10 mg/kg (body weight) in a single dose or about 200 mg to about 1200 mg in a single dose can be intravenously administered (for example, by intravenous infusion) over about 30 minutes to about 60 minutes or about 60 minutes or longer, at intervals of 2 to 4 weeks. Here, the dose per administration in terms of body weight is, for example, 1 mg/kg, 2 mg/kg, 3 mg/kg, 4 mg/kg, 5 mg/kg, 6 mg/kg, 7 mg/kg, 8 mg/kg, 9 mg/kg, or 10 mg/kg, and the dose per administration is, for example, 200 mg, 240 mg, 250 mg, 280 mg, 300 mg, 320 mg, 350 mg, 360 mg, 400 mg, 420 mg, 450 mg, 480 mg, 500 mg, 540 mg, 560 mg, 600 mg, 640 mg, 700 mg, 720 mg, 750 mg, 800 mg, 840 mg, 900 mg, 1000 mg, 1080 mg, 1100 mg, 1120 mg, or 1200 mg. In addition, the administration interval is, for example, 2 weeks, 3 weeks, or 4 weeks, and the single administration period is, for example, about 30 minutes to about 60 minutes, or about 60 minutes or more.

When the active ingredient is Nivolumab, which is an anti-PD-1 antibody, it is administered in the following dosage and administration. That is, to a malignant melanoma patient, 3 mg/kg (body weight) of Nivolumab in a single dose is administered by intravenous infusion at intervals of 2 weeks, or 2 mg/kg (body weight) of the same in a single dose is administered by intravenous infusion at intervals of 3 weeks. Alternatively, 240 mg of the same in a single dose is administered by intravenous infusion at intervals of 2 weeks, or 480 mg of the same in a single dose is administered by intravenous infusion at intervals of 4 weeks. To each patient of non-small cell lung cancer, renal cell cancer, classical Hodgkin lymphoma, head and neck cancer, gastric cancer, or malignant pleural mesothelioma, 3 mg/kg (body weight) of Nivolumab in a single dose is administered by intravenous infusion at intervals of 2 weeks. In addition, regarding another dosage and administration, for example, to each patient of malignant melanoma, non-small cell lung cancer, renal cell cancer, classical Hodgkin lymphoma, head and neck cancer, urothelial cancer, MSI-H or dMMR-positive colon cancer (also included are pediatric patients over the age of 12), gastric cancer, esophageal cancer, hepatocellular cancer, small cell lung cancer, and malignant pleural mesothelioma, 240 mg of Nivolumab in a single dose is administered by intravenous infusion at intervals of 2 weeks, or 480 mg of Nivolumab in a single dose is administered by intravenous infusion at intervals of 4 weeks. Furthermore, regarding still another dosage and administration, for example, to each patient of malignant melanoma, in combination with Ipilimumab, 1 mg/kg (body weight) of Nivolumab in a single dose may be administered by intravenous infusion 4 times at intervals of 3 weeks, and then 3 mg/kg (body weight) of Nivolumab in a single dose may be administered thereto by intravenous infusion at intervals of 2 weeks; or 80 mg of Nivolumab in a single dose may be administered by intravenous infusion 4 times at intervals of 3 weeks, and then 240 mg of Nivolumab in a single dose may be administered by intravenous infusion at intervals of 2 weeks or 480 mg of Nivolumab in a single dose may be administered by intravenous infusion at intervals of 4 weeks. Furthermore, for example, to each patient of renal cell cancer or colon cancer, 240 mg of Nivolumab in a single dose may be administered, in combination with Ipilimumab, by intravenous infusion 4 times at intervals of 3 week, followed by administration of 240 mg of Nivolumab in a single dose by intravenous infusion at intervals of 2 weeks, or followed by administration of 480 mg of Nivolumab in a single dose by intravenous infusion at intervals of 4 weeks.

In the case of Cemiplimab, which is also an anti-PD-1 antibody, 350 mg of the same in a single dose is administered at intervals of 3 weeks.

In the case of Pembrolizumab, which is also an anti-PD-1 antibody, it is administered in the following dosage and administration. That is, to each patient of malignant melanoma, non-small cell lung cancer, classical Hodgkin lymphoma, head and neck cancer, MSI-H or dMMR-positive solid cancer or colon cancer, urothelial cancer, cervical cancer, primary mediastinal B-cell lymphoma, hepatocellular cancer, gastric cancer, and Merkel cell cancer, 200 mg of Pembrolizumab in a single dose is administered by intravenous infusion at intervals of 3 weeks, or 400 mg of Pembrolizumab in a single dose is administered by intravenous infusion at intervals of 6 weeks. Furthermore, regarding another dosage and administration, for example, to each pediatric patient over the age of 2 or more with classical Hodgkin lymphoma, MSI-H or dMMR-positive solid cancer or colon cancer, and primary mediastinal B-cell lymphoma, 2 mg/kg (body weight) of Pembrolizumab in a single dose (up to 200 mg in a single dose) is administered by intravenous infusion at intervals of 3 weeks.

In the case of Avelumab containing an anti-PD-L1 antibody as its active ingredient, to each patient with Merkel cell cancer and urothelial cancer, 10 mg/kg (body weight) of Avelumab in a single dose is administered by intravenous infusion at intervals of 2 weeks. In the case of Atezolizumab, which is also a PD-L1 antibody, to each patient with non-small cell lung cancer, urothelial cancer and hepatocellular cancer, 1200 mg of Atezolizumab in a single dose is administered by intravenous infusion at intervals of 3 weeks, to a patient with triple negative breast cancer, 840 mg of Atezolizumab in a single dose is administered, in combination with paclitaxel, by intravenous infusion at intervals of 2 weeks. Furthermore, in the case of Durvalumab, which is also a PD-L1 antibody, to each patient with non-small cell lung cancer and urothelial cancer, 10 mg/kg (body weight) in a single dose of Durvalumab is administered by intravenous infusion at intervals of 2 weeks, and to a patient with advanced small cell lung cancer, 1500 mg of Durvalumab in a single dose is administered by intravenous infusion at intervals of 4 weeks.

In the case of Ipilimumab, which is an anti-CTLA-4 antibody, 3 mg/kg (body weight) of Ipilimumab is administered, alone or in combination with Nivolumab, by intravenous infusion once a day 4 times at intervals of 3 weeks to each patient with malignant melanoma; 1 mg/kg (body weight) of Ipilimumab is administered in combination with Nivolumab by intravenous infusion once a day 4 times at intervals of 3 weeks to each patient with renal cell cancer and MSI-H or dMMR-positive colon cancer; and 1 mg/kg (body weight) of Ipilimumab in a single dose is administered by intravenous infusion at intervals of 6 weeks to each patient with non-small cell lung cancer.

In the present invention, the dosage and administration can also be used in the treatment method of the present invention.

As an administration form of intravenous administration of the immune checkpoint inhibitory substance of the present invention, intravenous infusion is preferable.

<u>(3) Combination therapy in which molecular target drug for example, Bevacizumab,</u>

<u>Cetuximab) is added to FOLFOX therapy (example of standard therapy)</u>

The "standard therapy" in the present invention is a treatment whose therapeutic effect and safety have been confirmed by the results of many clinical trials and which is most recommended on the basis of scientific evidence. The "FOLFOX therapy" in the present invention is a cancer treatment method by a combination of three agents of Oxaliplatin, Levofolinate calcium (hereinafter, abbreviated as "Levofolinate"), and Fluorouracil. In one embodiment, the "FOLFOX therapy" includes (1a) intravenous administration of 85 mg/m$^2$ to 130 mg/m$^2$ of Oxaliplatin, (1b) intravenous administration of 100 mg/m$^2$ to 200 mg/m$^2$ of Levofolinate, (1c) rapid intravenous administration of 400 mg/m$^2$ of Fluorouracil, and (1d) intravenous administration of 600 mg/m$^2$ to 2400 mg/m$^2$ of Fluorouracil, and the administrations in (1a), (1b), (1c), and (1d) above in series are performed at intervals of 2 weeks. The doses in this therapeutic method are all doses in terms of body surface area per administration. Multiple formulations of the FOLFOX therapy are known which depend on the doses of the three agents to be administered and the administration method. As aspects thereof, for example, the rapid intravenous administration of Fluorouracil in (1c) may not be performed; the dose of Oxaliplatin in (1a) may be 85 mg/m$^2$, 100 mg/m$^2$, 130 mg/m$^2$, or any in a range of 85 mg/m$^2$ to 130 mg/m$^2$; the dose of Levofolinate in (1b) may be 100 mg/m$^2$, 200 mg/m$^2$, or any in a range of 100 mg/m$^2$ to 200 mg/m$^2$; and the dose of Fluorouracil continuously administered intravenously in (1d) may be 600 mg/m$^2$, 1500 mg/m$^2$, 2400 mg/m$^2$, or any in a range of 600 mg/m$^2$ to 2400 mg/m$^2$.

In one embodiment, in the rapid intravenous administration, the agent is administered within 15 minutes.

In one embodiment, for example, the FOLFOX therapy, referred to as FOLFOX4 therapy, includes: (1a) intravenously administering 85 mg/m$^2$ of Oxaliplatin and (1b) 100 mg/m$^2$ of Levofolinate over 2 hours; (1c) rapidly intravenously administering 400 mg/m$^2$ of Fluorouracil after the completion of the administration of Oxaliplatin of (1a) and Levofolinate of (1b); (1d) intravenously administering additional 600 mg/m$^2$ of Fluorouracil over 22 hours after the completion of the administration of Fluorouracil of (1c); (1e) further intravenously administering additional 100 mg/m$^2$ of Levofolinate over 2 hours; (1f) rapidly intravenously administering 400 mg/m$^2$ of Fluorouracil after the completion of the administration of Levofolinate of (1e); and (1g) further intravenously administering 600 mg/m$^2$ of Fluorouracil over 22 hours after the completion of the administration of Fluorouracil of (1f), wherein the administrations in (1a), (1b), (1c), (1d), (1e), (1f), and (1g) above in series are performed at intervals of 2 weeks. The doses in this therapeutic method are all doses in terms of body surface area per administration.

In another embodiment, the FOLFOX therapy, referred to as mFOLFOX6 therapy, includes: (1a) intravenously administering 85 mg/m$^2$ of Oxaliplatin and (1b) 200 mg/m$^2$ of Levofolinate over 2 hours; (1c) rapidly intravenously administering 400 mg/m$^2$ of Fluorouracil after the completion of the administration of Oxaliplatin of (1a) and Levofolinate of (1b); and (1d) further intravenously administering 2400 mg/m$^2$ of Fluorouracil over 46 hours after the completion of the administration of Fluorouracil of (1c), wherein the administrations in (1a), (1b), (1c), and (1d) above in series are performed at intervals of 2 weeks. The doses in this therapeutic method are all doses in terms of body surface area per administration.

In the present specification, the FOLFOX therapy encompasses all methods selected according to the administration method of each drug, for example, methods such as the FOLFOX4 therapy and the mFOLFOX6 therapy.

In addition, the interval for the administrations in series in the combination therapy in which the molecularly target drug is added to the FOLFOX therapy may be set to an interval of 2 weeks, or may be temporarily or continuously set to an interval of 3 weeks or more (for example, an interval of 3 weeks or 4 weeks) according to the degree of side effect onset in the patient. Withdrawal, reduction, and restart of the combination therapy in which Bevacizumab or Cetuximab is added to the FOLFOX therapy are performed by the judgment of the principal investigator or the co-investigator with reference to the latest package insert.

Examples of the molecular target drug include an anti-EGFR antibody (for example, Cetuximab, Panitumumab) and an anti-VEGF antibody (for example, Bevacizumab). One kind of the molecular target drug may be used singly, or two or more kinds of the same may be used in combination. In one embodiment, the presence or absence of a RAS gene (KRAS/NRAS gene) mutation is taken into consideration when a patient to whom the molecular target drug is applied is selected. The anti-EGFR antibody (for example, Cetuximab, Panitumumab), in one embodiment, is used for RAS wild-type colon cancer. The anti-VEGF antibody (for example, Bevacizumab), in one embodiment, is used for RAS mutated-type colon cancer.

In one embodiment, 5 mg/kg (body weight) of Bevacizumab, as a starting dose, is administered by intravenous infusion to a patient with RAS mutated-type cancer over 90 minutes. If the first dose is well tolerated, the second dose can be administered over 60 minutes. If the second dose is also well tolerated, the subsequent dose can be administered over 30 minutes. The administration interval can be 2 weeks or longer (for example, 2 weeks, 3 weeks, or 4 weeks).

In one embodiment, 400 mg/m$^2$ (body surface area) of Cetuximab, as a starting dose, is administered by intravenous infusion to a patient with RAS wild-type colon cancer over 2 hours. As the second and subsequent doses, 250 mg/m$^2$ is administered by intravenous infusion over 1 hour once a week or at intervals of 2 weeks. Alternatively, if necessary, Cetuximab of 500 mg/m$^2$ can be administered by intravenous infusion over 2 hours once a two weeks, i.e., at intervals of 2 weeks, and the dose is appropriately reduced depending on the condition of the patient.

In one embodiment, 6 mg/kg (body weight) of Panitumumab in a single dose is administered by intravenous infusion to a patient with RAS wild-type colon cancer over 60 minutes or more once a two weeks. The dose is appropriately reduced according to the condition of the patient.

(4) Use in combination with combination therapy in which molecular target drug (for

example, Bevacizumab, Cetuximab) is added to FOLFOX therapy

In one embodiment, the CD47 inhibitory substance in combination with a combination therapy in which a molecular target drug (for example, Bevacizumab, Cetuximab) is added to the FOLFOX therapy is administered in the dosage and administration described in "(1) CD47 inhibitory substance" described above. Magrolimab, an example of the CD47 inhibitory substance, is administered in the following manner in one embodiment: 1 mg/kg of Magrolimab, as an initial dose, is intravenously administered over 3 hours ($\pm$ 30 minutes), and thereafter, 20 mg/kg or 30 mg/kg in a single dose is intravenously administered at intervals of 1 week or 2 weeks. In one embodiment, 1 mg/kg of Magrolimab, as an initial dose, is intravenously administered over 3 hours ($\pm$ 30 minutes) on Day 1 of the first cycle, and then 20 mg/kg or 30 mg/kg of the same in a single dose is intravenously administered over 2 hours ($\pm$ 30 minutes) at intervals of 1 week in the first cycle and at intervals of 2 weeks in the second cycle or thereafter. According to the degree of side effect onset in the patient, the dose of Magrolimab may be reduced one level by one level, or the administration itself may be stopped. Also, if criteria for restart are met, the administration of Magrolimab can be restarted, in which case the administration can be restarted with the dose of Magrolimab reduced by the principal investigator or co-investigator's discretion. In one embodiment, when Magrolimab as a CD47 inhibitory substance is administered at a starting dose of 30 mg/kg, the dose is 20 mg/kg after 1-step weight reduction and 15 mg/kg after 2-step weight reduction. When Magrolimab as a CD47 inhibitory substance is administered at a starting dose of 20 mg/kg, the dose is 15 mg/kg after weight reduction.

In one embodiment, the immune checkpoint inhibitor in combination with a combination therapy in which a molecular target drug (for example, Bevacizumab, Cetuximab) is added to the FOLFOX therapy is administered in the dosage and administration described in "(2) Immune checkpoint inhibitory substance" described above. In one embodiment, regarding the administration of Nivolumab as an immune checkpoint inhibitor, 240 mg of Nivolumab in a single dose is administered by intravenous infusion at intervals of 2 weeks; 360 mg of the same in a single dose is administered by intravenous infusion at intervals of 3 weeks; or 480 mg of the same in a single dose is administered by intravenous infusion at intervals of 4 weeks. Preferably, 480 mg of Nivolumab in a single dose is administered by intravenous infusion at intervals of 4 weeks. More preferably, 480 mg of Nivolumab in a single dose is administered by intravenous infusion over about 30 minutes at intervals of 4 weeks. The administration of Nivolumab itself may be stopped according to the degree of side effect onset in the patient. In addition, if the criteria for restart are satisfied, the administration of Nivolumab can be restarted.

In the treatment method of the present invention, when a combination therapy in which a CD47 inhibitory substance (preferably Magrolimab) and an immune checkpoint inhibitor (preferably an anti-PD-1 antibody (preferably Nivolumab)), and a combination therapy in which a molecular target drug (preferably Bevacizumab or Cetuximab) are added to FOLFOX therapy, are used in combination (for example, the administration is performed on the same day or is started on the same day), in one embodiment, the CD47 inhibitory substance and the immune checkpoint inhibitor are administered first. In one embodiment, the CD47 inhibitory substance and the immune checkpoint inhibitor are administered, and then, the combination therapy with bevacizumab or cetuximab added to the FOLFOX therapy is performed.

(5) FFX therapy, or dose-reduced regimen thereof (another example of standard therapy)

The "FFX therapy " in the present invention is a cancer treatment method using a combination of four agents of Oxaliplatin, Irinotecan hydrochloride hydrate (hereinafter abbreviated as "Irinotecan"), Levofolinate calcium (hereinafter, abbreviated as "Levofolinate"), and Fluorouracil. A recommended dosage and administration include: (2a) intra-venously administering 85 mg/m$^2$ of Oxaliplatin over 2 hours, and thereafter; (2b) intravenously administering 200 mg/m$^2$ of Levofolinate over 2 hours; (2c) intravenously administering 180 mg/m$^2$ of Irinotecan over 1.5 hours, the administration being started 30 minutes after the start of the administration of Levofolinate of the above (2b); (2d) rapidly intravenously administering additional 400 mg/m$^2$ of Fluorouracil after the completion of the administration of Levofolinate of the above (2b); and (2e) further intravenously administering additional 2400 mg/m$^2$ of Fluorouracil over 46 hours after the completion of the administration of Fluorouracil of the above (2d), wherein the series of administrations in (2a), (2b), (2c), (2d), and (2e) is performed at intervals of 2 weeks. The doses in this therapeutic method are all doses in terms of body surface area per administration.

The "dose-reduced regimen" of the FFX therapy is a prescription for reducing the dose of any one of the four agents to be administered in the FFX therapy in the first and subsequent administration, or stopping the administration itself; or reducing the dose in any administration in the second and subsequent cycles, or stopping the administration of any one of the four agents, according to the degree of side effect onset observed in any administration in the first and subsequent cycles. As aspects thereof, for example, in the first and subsequent administration, the rapid intravenous administration of Fluorouracil in the above (2d) may not be performed; the dose of Oxaliplatin in the above (2a) may be 50 mg/m$^2$, 65 mg/m$^2$, or any in a range of 50 to 85 mg/m$^2$; the dose of Irinotecan in the above (2c) may be 90 mg/m$^2$, 120 mg/m$^2$, 150 mg/m$^2$, or any in a range of 90 to 180 mg/m$^2$; and the dose of Fluorouracil continuously administered intravenously in the above (2e) may be 1200 mg/m$^2$, 1800 mg/m$^2$, or any in a range of 1200 to 2400 mg/m$^2$.

In addition, as another aspect of the dose-reduced regimen, in any administration in the second and subsequent cycles in the FFX therapy, the rapid intravenous administration of Fluorouracil in the above (2d) may be stopped according to the degree of side effect onset in the patient; the dose of Oxaliplatin in the above (2a) may be reduced to 50 mg/m$^2$, 65 mg/m$^2$, or any in a range of 50 to 85 mg/m$^2$, or the administration of Oxaliplatin may be stopped, according to the degree of side effect onset in the patient; the dose of Irinotecan in the above (2c) may be reduced to 90 mg/m$^2$, 120 mg/m$^2$, 150 mg/m$^2$, or any in a range of 90 to 180 mg/m$^2$, or the administration of Irinotecan may be stopped, according to the degree of side effect onset in the patient; and the dose of Fluorouracil that is continuously administered intravenously in the above (2e) may be reduced to 1200 mg/m$^2$, 1800 mg/m$^2$, or any in a range of 1200 to 2400 mg/m$^2$, or the administration of Fluorouracil may be stopped, according to the degree of side effect onset in the patient.

In another aspect of this dose-reduced regimen, which is recognized as the modified FOLFIRINOX therapy (mFFX therapy), a recommended dosage and administration include includes: (2a) intravenously administering 85 mg/m$^2$ of Oxaliplatin over 2 hours, and thereafter; (2b) intravenously administering 200 mg/m$^2$ of Levofolinate over 2 hours; (2c) intravenously administering 150 mg/m$^2$ of Irinotecan over 1.5 hours, the administration being started 30 minutes after the start of the administration of Levofolinate of the above (2b); and (2e) further intravenously administering additional 2400 mg/m$^2$ of Fluorouracil over 46 hours after the completion of the administration of Levofolinate of the above (2b), wherein the administrations (2a), (2b), (2c), and (2e) above in series are performed at intervals of 2 weeks. The doses in this therapeutic method are all doses in terms of body surface area per administration.

As another dose-reduced regimen of the mFFX therapy, for example, in the first and subsequent administration, the dose of Oxaliplatin in the above (2a) may be 50 mg/m$^2$, 65 mg/m$^2$, or any in a range of 50 to 85 mg/m$^2$; the dose of Irinotecan in the above (2c) may be 120 mg/m$^2$, 140 mg/m$^2$, or any in a range of 120 to 140 mg/m$^2$; and the dose of Fluorouracil continuously administered intravenously in the above (2e) may be 1200 mg/m$^2$, 1800 mg/m$^2$, 2400 mg/m$^2$, or any in a range of 1200 to 2400 mg/m$^2$.

In addition, in any administration in the second and subsequent cycles in the mFFX therapy, the dose of Oxaliplatin in the above (2a) may be reduced to 50 mg/m$^2$, 65 mg/m$^2$, or any in a range of 50 to 85 mg/m$^2$, or the administration of Oxaliplatin may be stopped, according to the degree of side effect onset in the patient; the dose of Irinotecan in the above (2c) may be reduced to 90 mg/m$^2$, 120 mg/m$^2$, or any in a range of 90 to 150 mg/m$^2$, or the administration of Irinotecan may be stopped, according to the degree of side effect onset in the patient; and the dose of Fluorouracil that is continuously administered intravenously in the above (2e) may be reduced to 1200 mg/m$^2$, 1800 mg/m$^2$, or any in a range of 1200 to 2400 mg/m$^2$, or the administration of Fluorouracil may be stopped, according to the degree of side effect onset in the patient.

In addition, the interval for the administrations in series in the FFX therapy or a dose-reduced regimen of the same may be temporarily or continuously set to an interval of 3 weeks or 4 weeks according to the degree of side effect onset in the patient. Withdrawal, reduction, and restart of the FFX therapy or a dose-reduced regimen of the same (for example, mFFX therapy) are performed by the judgment of the principal investigator or the co-investigator with reference to the latest package insert.

(6) Use in combination with FFX therapy or dose-reduced regimen thereof

In one embodiment, the CD47 inhibitory substance in combination with the FFX therapy or its dose-reduced regimen (for example, mFFX therapy) is administered in the dosage and administration described in "(1) CD47 inhibitory substance" described above. Magrolimab, an example of the CD47 inhibitory substance, is administered in the following manner in one embodiment: 1 mg/kg of Magrolimab, as an initial dose, is intravenously administered over 3 hours ($\pm$ 30 minutes), and thereafter, 20 mg/kg or 30 mg/kg in a single dose is intravenously administered at intervals of 1 week or 2 weeks. In one embodiment, 1 mg/kg of Magrolimab, as an initial dose, is intravenously administered over 3 hours ($\pm$ 30 minutes) on Day 1 of the first cycle, and then 20 mg/kg or 30 mg/kg of the same in a single dose is intravenously administered over 2 hours ($\pm$ 30 minutes) at intervals of 1 week in the first cycle and at intervals of 2 weeks in the second cycle or thereafter. According to the degree of side effect onset in the patient, the dose of Magrolimab may be reduced one level by one level, or the administration itself may be stopped. Also, if criteria for restart are met, the administration of Magrolimab can be restarted, in which case the administration can be restarted with the dose of Magrolimab reduced by the principal investigator or co-investigator's discretion. In one embodiment, when Magrolimab as a CD47 inhibitory substance is administered at a starting dose of 30 mg/kg, the dose is 20 mg/kg after 1-step weight reduction and 15 mg/kg after 2-step weight reduction. When Magrolimab as a CD47 inhibitory substance is administered at a starting dose of 20 mg/kg, the dose is 15 mg/kg after weight reduction.

In one embodiment, the immune checkpoint inhibitor in combination with the FFX therapy or its dose-reduced regimen is administered in the dosage and administration described in "(2) Immune checkpoint inhibitory substance" described above. In one embodiment, regarding the administration of Nivolumab as an immune checkpoint inhibitor, 240 mg of Nivolumab in a single dose is administered by intravenous infusion at intervals of 2 weeks; 360 mg of the same in a single dose is administered by intravenous infusion at intervals of 3 weeks; or 480 mg of the same in a single dose is administered by intravenous infusion at intervals of 4 weeks. Preferably, 480 mg of Nivolumab in a single dose is administered by intravenous infusion at intervals of 4 weeks. More preferably, 480 mg of Nivolumab in a single dose is administered by intravenous infusion over about 30 minutes at intervals of 4 weeks. The administration of Nivolumab itself may be stopped according to the degree of side effect onset in the patient. In addition, if the criteria for restart are satisfied, the administration of Nivolumab can be restarted.

In the treatment method of the present invention, when a combination therapy in which a CD47 inhibitory substance (preferably Magrolimab) and an immune checkpoint inhibitor (preferably an anti-PD-1 antibody (preferably Nivolumab)), and the FFX therapy or its dose-reduced regimen (for example, the mFFX therapy), are used in combination (for example, the administration is performed on the same day or is started on the same day), in one embodiment, the CD47 inhibitory substance and the immune checkpoint inhibitor are administered first. In one embodiment, the CD47 inhibitory substance and the immune checkpoint inhibitor are administered, and then, the FFX therapy or its dose-reduced regimen (for example, the mFFX therapy) is performed.

[Therapy-applied diseases and patients]

Diseases to which the treatment method of the present invention is to be applied are cancer.

More specific examples of the cancer include leukemia (for example, acute myelogenous leukemia, chronic myelogenous leukemia, acute lymphocytic leukemia, chronic lymphocytic leukemia), malignant lymphoma (Hodgkin lymphoma, non-Hodgkin lymphoma (for example, adult T-cell leukemia, follicular lymphoma, diffuse large B-cell lymphoma)), multiple myeloma, myelodysplastic syndrome, head and neck cancer, esophageal cancer, esophageal adenocarcinoma, gastric cancer, esophagogastric junction cancer, duodenal cancer, colon cancer (for example, colorectal cancer), liver cancer (for example, hepatocellular cancer), gallbladder/bile duct cancer, biliary tract cancer, pancreatic cancer (for example, pancreatic ductal cancer, insulinoma, intraductal papillary mucinous tumor, pancreatic cancer having distant metastasis, pancreatic ductal cancer having distant metastasis), thyroid cancer, lung cancer (for example, non-small cell lung cancer (for example, squamous non-small cell lung cancer, non-squamous non-small cell lung cancer), small cell lung cancer), breast cancer, ovarian cancer (for example, serous ovarian cancer), cervical cancer, uterine body cancer, endometrial cancer, vaginal cancer, vulval cancer, renal cancer (for example, renal cell cancer), pelvis/ureter cancer, urothelial cancer (for example, bladder cancer, upper urinary tract cancer), penile cancer, prostate cancer, testicular tumor (for example, embryonic cell tumors), bone/soft tissue sarcoma, malignant bone tumor, skin cancer (for example, uveal malignant melanoma, malignant melanoma, Merkel cell carcinoma), thymoma, mesothelioma, malignant pleural mesothelioma, glioblastoma, hematologic cancer, and unknown primary cancer.

One aspect of the disease to be treated by the treatment method of the present invention is, for example, solid cancer. Examples of the solid cancer include head and neck cancer, esophageal cancer, esophageal adenocarcinoma, gastric cancer, esophagogastric junction cancer, duodenal cancer, colon cancer (for example, colorectal cancer), liver cancer (for example, hepatocellular cancer), gallbladder/bile duct cancer, biliary tract cancer, pancreatic cancer (for example, pancreatic ductal cancer, insulinoma, intraductal papillary mucinous tumor, pancreatic cancer having distant metastasis, pancreatic ductal cancer having distant metastasis), thyroid cancer, lung cancer (for example, non-small cell lung cancer (for example, squamous non-small cell lung cancer, non-squamous non-small cell lung cancer), small cell lung cancer), breast cancer, ovarian cancer (for example, serous ovarian cancer), cervical cancer, uterine body cancer, endometrial cancer, vaginal cancer, vulval cancer, renal cancer (for example, renal cell cancer), pelvis/ureter cancer, urothelial cancer (for example, bladder cancer, upper urinary tract cancer), penile cancer, prostate cancer, testicular tumor (for example, embryonic cell tumors), bone/soft tissue sarcoma, malignant bone tumor, skin cancer (for example, uveal malignant melanoma, malignant melanoma, Merkel cell carcinoma), thymoma, mesothelioma, malignant pleural mesothelioma, glioblastoma, and unknown primary cancer.

The disease to which the treatment method of the present invention is applied is preferably colon cancer or pancreatic cancer, and more preferably radically unresectable advanced or recurrent colon cancer, or pancreatic cancer having distant metastasis. More preferably, the disease is radically unresectable advanced or recurrent colorectal cancer or pancreatic ductal cancer having distant metastasis.

In the present specification, "treatment" of cancer encompasses, for example, a treatment performed to (i) reduce the proliferation of tumor cells, (ii) reduce symptoms resulting from cancer, (iii) improve the quality of life of a cancer patient, (iv) reduce the dose of other anticancer drugs or cancer treatment adjuvants that have already been administered, and/or (v) prolong the survival of a cancer patient, and "progression inhibition" of cancer means slowing the progression of cancer, stabilizing symptoms associated with cancer, and reversing the progression of symptoms. In addition, the "suppression of recurrence" of cancer means to prevent the recurrence of cancer in a patient whose cancer lesion has completely or substantially disappeared or removed by cancer treatment or resection surgery.

The treatment method of the present invention may be applied to the following cancer patient: (a) a patient treated with an anticancer drug whose effect has been insufficient or not effective enough, or a patient with exacerbation after treatment with an anticancer drug; (b) a patient with incurable or unresectable, metastatic, recurrent, refractory and/or distant metastatic cancer; (c) a patient with cancer having a Tumor Proportion Score (TPS) of 50% or more, 25% or more, 10% or more, 5% or more, or 1% or more; (d) a patient with cancer having a Combined Positive Score (CPS) of 20% or more, 10% or more, 5% or more, or 1% or more; (e) a patient with cancer having a deficient mismatch repair (dMMR) and/or MSI-H (Microsatellite Instability-High; or (f) a patient with cancer having a high Tumor Mutational Burden (TMB). On the other hand, the treatment method of the present invention may be more required to be applied to the following cancer patient: (g) a patient who has not been treated with an anticancer drug; (h) a patient with cancer having a TPS of less than 50%, less than 25%, less than 10%, less than 5%, or less than 1%; (i) a patient with cancer having a CPS of less than 20%, less than 10%, less than 5%, or less than 1%; (j) a patient with cancer having no dMMR and/or MSI-H or having MSI-L (Microsatellite instability-Low); or (k) a patient with cancer having a low TMB. In particular, the cancer patient for whom the treatment method of the present invention is required to be applied is, for example, a cancer patient who has not been treated with an anticancer drug and/or a patient with radically unresectable advanced or recurrent cancer, in particular, a patient with colon cancer or pancreatic cancer. In one embodiment, the patient is, for example, a patient who has not been treated for colon cancer or a patient who has not been treated for pancreatic cancer. Preferably, the patient is, for example, a patient who has not been treated with a systemic malignant tumor agent for colon cancer, or a patient who has not been treated with a systemic malignant tumor agent for pancreatic cancer. More preferably, the patient is, for example, a patient who has not been treated with a systemic anti-malignant tumor agent for radically unresectable advanced or recurrent colon cancer, or a patient who has not been treated with a systemic anti-malignant tumor agent for pancreatic cancer having distant metastasis.

As one aspect, the treatment method of the present invention is used as a primary treatment for a patient who has not been treated with a systemic anti-malignant tumor agent for radically unresectable advanced or recurrent colon cancer.

As one aspect, the treatment method of the present invention is used as a primary treatment for a patient who has not been treated with a systemic anti-malignant tumor agent for pancreatic cancer having distant metastasis.

As one aspect, the treatment method of the present invention is also applicable for treating metastatic cancer and suppressing cancer metastasis.

As one aspect, the treatment method of the present invention suppresses recurrence.

In the present invention, treatment means, as one aspect, causing at least one of reduction in tumor size, suppression of tumor growth (delay or stop), suppression of metastasis (delay or stop) of tumor, suppression of recurrence (prevention or delay), and alleviation of one or more symptoms associated with cancer.

The treatment method of the present invention, as one aspect, may be used in combination with other drugs (for example, known anticancer drugs, antiemetics) for (1) complementation and/or enhancement of therapeutic effects, (2) improvement of kinetics and absorption, reduction of dosage, and/or (3) reduction of side effects.

In the present specification, "about" means that the numerical value may change below or above the displayed numerical value by within 10%.

The present invention provides, for example, the following embodiments.

[1] An agent for suppressing progression of, suppressing recurrence of, and/or treating a solid cancer, comprising a CD47 inhibitory substance as an active ingredient, wherein the agent is administered in combination with a standard therapy and an immune checkpoint inhibitory substance.

[2] An agent for suppressing progression of, suppressing recurrence of, and/or treating a solid cancer, comprising an immune checkpoint inhibitory substance as an active ingredient, wherein the agent is administered in combination with a standard therapy and a CD47 inhibitory substance.

[3] The agent according to [1] or [2] above, wherein the standard therapy is a combination therapy in which a molecular target drug (preferably a VEGF inhibitor (preferably Bevacizumab) or an EGFR inhibitor (preferably Cetuximab or Panitumumab)) is added to FOLFOX therapy.

[4] The agent according to any one of [1] to [3] above, wherein the standard therapy is a combination therapy in which Bevacizumab or Cetuximab is added to FOLFOX therapy.

[5] The agent according to any one of [1] to [4] above, wherein the solid cancer is colon cancer.

[6] The agent according to [5] above, wherein the colon cancer is colorectal cancer.

[7] The agent according to [6] above, wherein the colorectal cancer is radically unresectable advanced or recurrent colorectal cancer.

[8] The agent according to any one of [1] to [7] above, wherein the agent is administered as a primary therapy to a patient who has not been treated with a systemic anti-malignant tumor agent against radically unresectable advanced or recurrent colorectal cancer.

[9] The agent according to any one of [1] to [8] above, wherein the CD47 inhibitory substance is an anti-CD47 antibody.

[10] The agent according to [9] above, wherein the anti-CD47 antibody is Magrolimab.

[11] The agent according to [10] above, wherein 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations.

[12] The agent according to [10] or [11] above, wherein 1 mg/kg (body weight) of Magrolimab is intravenously administered for a first administration, and 20 mg/kg (body weight) of the same in a single dose is intravenously administered at intervals of 1 week or 2 weeks for a second and subsequent administrations (preferably, 1 mg/kg (body weight) of Magrolimab is intravenously administered for the first administration, and 20 mg/kg (body weight) of the same in a single dose is administered at intervals of 1 week in a first cycle and at intervals of 2 weeks in a second and subsequent cycles for the second and subsequent administrations).

[13] The agent according to [10] or [11] above, wherein 1 mg/kg (body weight) of Magrolimab is intravenously administered for a first administration, and 30 mg/kg (body weight) of the same in a single dose is intravenously administered at intervals of 1 week or 2 weeks for a second and subsequent administrations (preferably, 1 mg/kg (body weight) of Magrolimab is intravenously administered for the first administration, and 30 mg/kg (body weight) of the same is administered at intervals of 1 week in a first cycle and at intervals of 2 weeks in a second and subsequent cycles for the second and subsequent administrations).

[14] The agent according to any one of [1] to [13] above, wherein the immune checkpoint inhibitory substance is an anti-PD-1 antibody, an anti-PD-L1 antibody, or an anti-CTLA-4 antibody.

[15] The agent according to [14] above, wherein the immune checkpoint inhibitory substance is an anti-PD-1 antibody.

[16] The agent according to [14] or [15] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, Retifanlimab, Balstilimab, CS1003, Serplulimab, BAT-1306, AK105, AK103, BI 754091, LZM009, CMAB819, Sym021, Geptanolimab, SSI-361, JY034, HX008, ISU106, Budigalimab, Prolgolimab, Sasanlimab, CX-188, Cetrelimab, or Zimberelimab.

[17] The agent according to [14] above, wherein the immune checkpoint inhibitory substance is an anti-PD-L1 antibody, and the anti-PD-L1 antibody is Atezolizumab, Avelumab, Durvalumab, BMS-936559, STI-1014, Envafolimab, Lodapolimab, HLX20, SHR-1316, CS1001, MSB2311, BGB-A333, KL-A167, CK-301, AK106, AK104, ZKAB001, FAZ053, CBT-502, JS003, or CX-072.

[18] The agent according to [14] above, wherein the immune checkpoint inhibitory substance is an anti-CTLA-4 antibody, and the anti-CTLA-4 antibody is Ipilimumab, AGEN1884, or Tremelimumab.

[19] The agent according to [15] above, wherein the anti-PD-1 antibody is Nivolumab.

[20] The agent according to [15] above, wherein the anti-PD-1 antibody is Pembrolizumab.

[21] The agent according to [15] above, wherein the anti-PD-1 antibody is Cemiplimab.

[22] The agent according to [17] above, wherein the anti-PD-L1 antibody is Avelumab.

[23] The agent according to [17] above, wherein the anti-PD-L1 antibody is Atezolizumab.

[24] The agent according to [17] above, wherein the anti-PD-L1 antibody is Durvalumab.

[25] The agent according to [18] above, wherein the anti-CTLA-4 antibody is Ipilimumab.

[26] The agent according to [19] above, wherein 3 mg/kg (body weight) in a single dose, or 240 mg in a single does, of Nivolumab is administered at intervals of 2 weeks; 360 mg of Nivolumab in a single dose is administered at intervals of 3 weeks; or 480 mg of Nivolumab in a single dose is administered at intervals of 4 weeks (preferably, 240 mg of Nivolumab in a single dose is administered at intervals of 2 weeks; 360 mg of Nivolumab in a single dose is administered at intervals of 3 weeks; or 480 mg of Nivolumab in a single dose is administered at intervals of 4 weeks).

[27] The agent according to [19] above, wherein 480 mg of Nivolumab in a single dose is intravenously administered at intervals of 4 weeks.

[28] The agent according to [19] above, wherein 480 mg of Nivolumab in a single dose is intravenously administered over 30 minutes at intervals of 4 weeks.

[29] The agent according to [20] above, wherein 2 mg/kg (body weight) of Pembrolizumab in a single dose, or 200 mg of Pembrolizumab in a single dose, is administered at intervals of 3 weeks, or 400 mg of Pembrolizumab in a single dose is administered at intervals of 6 weeks.

[30] The agent according to [21] above, wherein 350 mg of Cemiplimab in a single dose is administered at intervals of 3 weeks.

[31] The agent according to [22] above, wherein 10 mg/kg (body weight) of Avelumab in a single dose is administered at intervals of 2 weeks.

[32] The agent according to [23] above, wherein 1200 mg of Atezolizumab in a single dose is administered at intervals of 3 weeks.

[33] The agent according to [24] above, wherein 10 mg/kg (body weight) of Durvalumab in a single dose is administered at intervals of 2 weeks, or 1500 mg of Durvalumab in a single dose is intravenously administered 4 times at intervals of 4 weeks.

[34] The agent according to [25] above, wherein 3 mg/kg (body weight) of Ipilimumab in a single dose, or 1 mg/kg (body weight) of Ipilimumab in a single dose, is intravenously administered 4 times at intervals of 3 weeks, or 1 mg/kg (body weight) of Ipilimumab in a single dose is administered at intervals of 6 weeks

[35] The agent according to any one of [4] to [34] above, wherein the combination therapy in which Bevacizumab or Cetuximab is added to FOLFOX therapy is a therapy in which (1A-1) Bevacizumab or (1A-2) Cetuximab, (1B) Oxaliplatin, (1C) Levofolinate calcium, and (1D) Fluorouracil are administered in combination.

[36] The agent according to any one of [4] to [35] above, wherein the combination therapy in which Bevacizumab or Cetuximab is added to FOLFOX therapy is a therapy in which (1A-1) Bevacizumab, (1B) Oxaliplatin, (1C) Levofolinate calcium, and (1D) Fluorouracil are administered in combination.

[37] The agent according to any one of [4] to [35] above, wherein the combination therapy in which Bevacizumab or Cetuximab is added to FOLFOX therapy is a therapy in which (1A-2) Cetuximab, (1B) Oxaliplatin, (1C) Levofolinate calcium, and (1D) Fluorouracil are administered in combination.

[38] The agent according to any one of [4] to [35] above, wherein the combination therapy in which Bevacizumab or Cetuximab is added to FOLFOX therapy is a therapy that includes:

(1x) intravenously administering 5 mg/kg (body weight) of Bevacizumab, or intravenously administering 400 mg/m$^2$ (body surface area) of Cetuximab for a first administration and 250 mg/m$^2$ (body surface area) of Cetuximab for a second and subsequent administrations;

(1a) intravenously administering 85 mg/m$^2$ (body surface area) of Oxaliplatin;

(1b) intravenously administering 200 mg/m$^2$ (body surface area) of Levofolinate calcium; (1c) rapidly intravenously administering 400 mg/m$^2$ (body surface area) of Fluorouracil; and (1d) further continuously intravenously administering 2400 mg/m$^2$ (body surface area) of Fluorouracil after completion of the administration of Fluorouracil in (1c) above.

[39] The agent according to any one of [4] to [36] and [38] above, wherein the combination therapy in which Bevacizumab or Cetuximab is added to FOLFOX therapy is a therapy that includes:

> (1x) intravenously administering 5 mg/kg (body weight) of Bevacizumab;
> (1a) intravenously administering 85 mg/m$^2$ (body surface area) of Oxaliplatin;
> (1b) intravenously administering 200 mg/m$^2$ (body surface area) of Levofolinate calcium;
> (1c) rapidly intravenously administering 400 mg/m$^2$ (body surface area) of Fluorouracil; and
> (1d) further continuously intravenously administering 2400 mg/m$^2$ (body surface area) of Fluorouracil after completion of the administration of Fluorouracil in (1c) above.

[40] The agent according to any one of [4] to [35], [37], and [38] above, wherein the combination therapy in which Bevacizumab or Cetuximab is added to FOLFOX therapy is a therapy that includes:

> (1x) intravenously administering 400 mg/m$^2$ (body surface area) of Cetuximab for a first administration, and 250 mg/m$^2$ (body surface area) of Cetuximab for a second and subsequent administrations;
> (1a) intravenously administering 85 mg/m$^2$ (body surface area) of Oxaliplatin;
> (1b) intravenously administering 200 mg/m$^2$ (body surface area) of Levofolinate calcium;
> (1c) rapidly intravenously administering 400 mg/m$^2$ (body surface area) of Fluorouracil; and
> (1d) further continuously intravenously administering 2400 mg/m$^2$ (body surface area) of Fluorouracil after completion of the administration of Fluorouracil in (1c) above.

[41] The agent according to any one of [4] to [35] and [38] above, wherein the combination therapy in which Bevacizumab or Cetuximab is added to FOLFOX therapy is a therapy that includes:

> (1x) intravenously administering 5 mg/kg (body weight) of Bevacizumab over 90 minutes, or intravenously administering 400 mg/m$^2$ (body surface area) of Cetuximab over 2 hours for a first administration, and 250 mg/m$^2$ (body surface area) of Cetuximab over 1 hour for a second and subsequent administrations;
> (1a) intravenously administering 85 mg/m$^2$ (body surface area) of Oxaliplatin over 2 hours;
> (1b) intravenously administering 200 mg/m$^2$ (body surface area) of Levofolinate calcium over 2 hours, simultaneously with the administration of Oxaliplatin in (1a) above;
> (1c) rapidly intravenously administering 400 mg/m$^2$ (body surface area) of Fluorouracil after completion of the administration of Levofolinate calcium in (1b) above; and
> (1d) further continuously intravenously administering 2400 mg/m$^2$ (body surface area) of Fluorouracil over 46 hours after completion of the administration of Fluorouracil in (1c) above,

wherein the series of administrations in (1x), (1a), (1b), (1c), and (1d) above is performed at intervals of 2 weeks.

[42] The agent according to any one of [4] to [36], [38], [39], and [41] above, wherein the combination therapy in which Bevacizumab or Cetuximab is added to FOLFOX therapy is a therapy that includes:

> (1x) intravenously administering 5 mg/kg (body weight) of Bevacizumab over 90 minutes;
> (1a) intravenously administering 85 mg/m$^2$ (body surface area) of Oxaliplatin over 2 hours;
> (1b) intravenously administering 200 mg/m$^2$ (body surface area) of Levofolinate calcium over 2 hours, simultaneously with the administration of Oxaliplatin in (1a) above;
> (1c) rapidly intravenously administering 400 mg/m$^2$ (body surface area) of Fluorouracil after completion of the administration of Levofolinate calcium in (1b) above; and
> (1d) further continuously intravenously administering 2400 mg/m$^2$ (body surface area) of Fluorouracil over 46 hours after completion of the administration of Fluorouracil in (1c) above,

wherein the series of administrations in (1x), (1a), (1b), (1c), and (1d) above is performed at intervals of 2 weeks.

[43] The agent according to any one of [4] to [35], [37], [38], [40], and [41] above, wherein the combination therapy in which Bevacizumab or Cetuximab is added to FOLFOX therapy is a therapy that includes:

> (1x) intravenously administering 400 mg/m$^2$ (body surface area) of Cetuximab over 2 hours for a first administration, and 250 mg/m$^2$ (body surface area) of Cetuximab over 1 hour for a second and subsequent administrations;
> (1a) intravenously administering 85 mg/m$^2$ (body surface area) of Oxaliplatin over 2 hours;
> (1b) intravenously administering 200 mg/m$^2$ (body surface area) of Levofolinate calcium over 2 hours, simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil over 46 hours after completion of the administration of Fluorouracil in (1c) above,

wherein a series of administrations in (1x), (1a), (1b), (1c), and (1d) above is performed at intervals of 2 weeks.

[44] The agent according to any one of [4] to [43] above, wherein the combination therapy in which Bevacizumab or Cetuximab is added to FOLFOX therapy, and the administration of the immune checkpoint inhibitory substance and the CD47 inhibitory substance, are started on the same day.

[45] An agent for suppressing progression of, suppressing recurrence of, and/or treating a cancer, comprising a CD47 inhibitory substance as an active ingredient, the agent being administered in combination with a combination therapy in which Bevacizumab or Cetuximab is added to FOLFOX therapy, and with an immune checkpoint inhibitory substance,
wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 5 mg/kg (body weight) of Bevacizumab (preferably over 90 minutes), or intravenously administering 400 mg/m$^2$ (body surface area) of Cetuximab (preferably over 2 hours) for the first administration, and 250 mg/m$^2$ (body surface area) of Cetuximab (preferably over 1 hour) for the second and subsequent administrations;

(1a) intravenously administering 85 mg/m$^2$ (body surface area) of Oxaliplatin (preferably over 2 hours);

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[46] An agent for suppressing progression of, suppressing recurrence of, and/or treating a cancer, comprising a CD47 inhibitory substance as an active ingredient, the agent being administered in combination with a combination therapy in which Bevacizumab or Cetuximab is added to FOLFOX therapy, and with an immune checkpoint inhibitory substance,
wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 5 mg/kg (body weight) of Bevacizumab (preferably over 90 minutes);

(1a) intravenously administering 85 mg/m$^2$ (body surface area) of Oxaliplatin (preferably over 2 hours);

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[47] An agent for suppressing progression of, suppressing recurrence of, and/or treating a cancer, comprising a CD47 inhibitory substance as an active ingredient, the agent being administered in combination with a combination therapy in which Bevacizumab or Cetuximab is added to FOLFOX therapy, and with an immune checkpoint inhibitory substance,

wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 400 mg/m$^2$ (body surface area) of Cetuximab (preferably over 2 hours) for a first administration, and 250 mg/m$^2$ (body surface area) of Cetuximab (preferably over 1 hour) for a second and subsequent administrations;

(1a) intravenously administering 85 mg/m$^2$ (body surface area) of Oxaliplatin over 2 hours;

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[48] An agent for suppressing progression of, suppressing recurrence of, and/or treating a cancer, comprising an immune checkpoint inhibitory substance as an active ingredient, the agent being administered in combination with a combination therapy in which Bevacizumab or Cetuximab is added to FOLFOX therapy, and with a CD47 inhibitory substance,

wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 5 mg/kg (body weight) of Bevacizumab (preferably over 90 minutes), or intravenously administering 400 mg/m$^2$ (body surface area) of Cetuximab (preferably over 2 hours) for the first administration, and 250 mg/m$^2$ (body surface area) of Cetuximab (preferably over 1 hour) for the second and subsequent administrations;

(1a) intravenously administering 85 mg/m$^2$ (body surface area) of Oxaliplatin over 2 hours;

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[49] An agent for suppressing progression of, suppressing recurrence of, and/or treating a cancer, comprising an immune checkpoint inhibitory substance as an active ingredient, the agent being administered in combination with a combination therapy in which Bevacizumab or Cetuximab is added to FOLFOX therapy, and with a CD47 inhibitory substance,

wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 5 mg/kg (body weight) of Bevacizumab (preferably over 90 minutes);

(1a) intravenously administering 85 mg/m$^2$ (body surface area) of Oxaliplatin (preferably over 2 hours);

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[50] An agent for suppressing progression of, suppressing recurrence of, and/or treating a cancer, comprising an immune checkpoint inhibitory substance as an active ingredient, the agent being administered in combination with a combination therapy in which Bevacizumab or Cetuximab is added to FOLFOX therapy, and with a CD47 inhibitory substance,

wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,

(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and

(iii) the combination therapy in which Bevacizumab or Cetuximab is added to the FOLFOX therapy includes:

(1x) intravenously administering 400 mg/m$^2$ (body surface area) of Cetuximab (preferably over 2 hours) for a first administration, and 250 mg/m$^2$ (body surface area) of Cetuximab (preferably over 1 hour) for a second and subsequent administrations;

(1a) intravenously administering 85 mg/m$^2$ (body surface area) of Oxaliplatin (preferably over 2 hours);

(1b) intravenously administering 200 mg/m$^2$ (body surface area) of Levofolinate calcium (preferably over 2 hours), simultaneously with the administration of Oxaliplatin in (1a) above;

(1c) rapidly intravenously administering 400 mg/m$^2$ (body surface area) of Fluorouracil after completion of the administration of Levofolinate calcium in (1b) above; and

(1d) further continuously intravenously administering 2400 mg/m$^2$ (body surface area) of Fluorouracil (preferably over 46 hours) after completion of the administration of Fluorouracil in (1c) above,

wherein a series of administrations in (1x), (1a), (1b), (1c), and (1d) above is performed at intervals of 2 weeks.

[51] The agent according to any one of [36], [39], [42], [46], and [49] above, wherein the cancer is a RAS mutated-type cancer.

[52] The agent according to any one of the items [37], [40], [43], [47], and [50] above, wherein the cancer is a RAS wild-type cancer.

[53] The agent according to any one of [45] to [52] above, wherein the cancer is radically unresectable advanced or recurrent colorectal cancer.

[54] The agent according to [53] above, wherein the agent is administered as a primary therapy to a patient who has not been treated with a systemic anti-malignant tumor agent against radically unresectable advanced or recurrent colorectal cancer.

[55] The agent according to any one of [45] to [54] above, wherein the combination therapy in which Bevacizumab or Cetuximab is added to FOLFOX therapy, and the administration of Magrolimab and Nivolumab, are started on the same day.

[56] The agent according to [1] or [2] above, wherein the standard therapy is FOLFIRINOX therapy or its dose-reduced regimen.

[57] The agent according to any one of [1], [2] above, and [56] above, wherein the solid cancer is pancreatic cancer.

[58] The agent according to [57] above, wherein the pancreatic cancer is pancreatic cancer having distant metastasis.

[59] The agent according to any one of [1], [2], [56], [57], and [58] above, wherein the agent is administered as a primary therapy to a patient who has not been treated with a systemic anti-malignant tumor agent against pancreatic cancer having distant metastasis.

[60] The agent according to any one of [1], [2], and [56] to [59] above, wherein the CD47 inhibitory substance is an anti-CD47 antibody.

[61] The agent according to [60] above, wherein the anti-CD47 antibody is Magrolimab.

[62] The agent according to [61] above, wherein 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations.

[63] The agent according to [61] or [62] above, wherein 1 mg/kg (body weight) of Magrolimab is intravenously administered for a first administration, and 20 mg/kg (body weight) of the same in a single dose is intravenously administered at intervals of 1 week or 2 weeks for a second and subsequent administrations (preferably, 1 mg/kg (body weight) of Magrolimab is intravenously administered for the first administration, and 20 mg/kg (body weight) of the same in a single dose is administered at intervals of 1 week in a first cycle and at intervals of 2 weeks in a second and subsequent cycles for the second and subsequent administrations).

[64] The agent according to [61] or [62] above, wherein 1 mg/kg (body weight) of Magrolimab is intravenously administered for a first administration, and 30 mg/kg (body weight) of the same in a single dose is intravenously administered at intervals of 1 week or 2 weeks for a second and subsequent administrations (preferably, 1 mg/kg (body weight) of Magrolimab is intravenously administered for the first administration, and 30 mg/kg (body weight) of the same in a single dose is administered at intervals of 1 week in a first cycle and at intervals of 2 weeks in a second and subsequent cycles for the second and subsequent administrations).

[65] The agent according to any one of [1], [2], and [56] to [64] above, wherein the immune checkpoint inhibitory substance is an anti-PD-1 antibody, an anti-PD-L1 antibody, or an anti-CTLA-4 antibody.

[66] The agent according to [65] above, wherein the immune checkpoint inhibitory substance is an anti-PD-1 antibody.

[67] The agent according to [65] or [66] above, wherein the anti-PD-1 antibody is Nivolumab, Cemiplimab, Pembrolizumab, Spartalizumab, Tislelizumab, AMP-514, Dostarlimab, Toripalimab, Camrelizumab, Genolimzumab, Sintilimab, STI-A1110, ENUM 388D4, ENUM 244C8, GLS010, Retifanlimab, Balstilimab, CS1003, Serplulimab, BAT-1306, AK105, AK103, BI 754091, LZM009, CMAB819, Sym021, Geptanolimab, SSI-361, JY034, HX008, ISU106, Budigalimab, Prolgolimab, Sasanlimab, CX-188, Cetrelimab, or Zimberelimab.

[68] The agent according to [67] above, wherein the immune checkpoint inhibitory substance is an anti-PD-L1 antibody, and the anti-PD-L1 antibody is Atezolizumab, Avelumab, Durvalumab, BMS-936559, STI-1014, Envafolimab, Lodapolimab, HLX20, SHR-1316, CS1001, MSB2311, BGB-A333, KL-A167, CK-301, AK106, AK104, ZKAB001, FAZ053, CBT-502, JS003, or CX-072.

[69] The agent according to [65] above, wherein the immune checkpoint inhibitory substance is an anti-CTLA-4 antibody, and the anti-CTLA-4 antibody is Ipilimumab, AGEN1884, or Tremelimumab.

[70] The agent according to [66] above, wherein the anti-PD-1 antibody is Nivolumab.

[71] The agent according to [66] above, wherein the anti-PD-1 antibody is Pembrolizumab.

[72] The agent according to [66] above, wherein the anti-PD-1 antibody is Cemiplimab.

[73] The agent according to [68] above, wherein the anti-PD-L1 antibody is Avelumab.

[74] The agent according to [68] above, wherein the anti-PD-L1 antibody is Atezolizumab.

[75] The agent according to [68] above, wherein the anti-PD-L1 antibody is Durvalumab.

[76] The agent according to [69] above, wherein the anti-CTLA-4 antibody is Ipilimumab.

[77] The agent according to [70] above, wherein 3 mg/kg (body weight) in a single dose, or 240 mg in a single dose, of Nivolumab is administered at intervals of 2 weeks; 360 mg of Nivolumab in a single dose is administered at intervals of 3 weeks; or 480 mg of Nivolumab in a single dose is administered at intervals of 4 weeks (preferably, 240 mg of Nivolumab in a single dose is administered at intervals of 2 weeks; 360 mg of Nivolumab in a single dose is administered at intervals of 3 weeks; or 480 mg of Nivolumab in a single dose is administered at intervals of 4 weeks).

[78] The agent according to [70] above, wherein 480 mg of Nivolumab in a single dose is intravenously administered at intervals of 4 weeks.

[79] The agent according to [70] above, wherein 480 mg of Nivolumab in a single dose is intravenously administered over about 30 minutes at intervals of 4 weeks.

[80] The agent according to [71] above, wherein 2 mg/kg (body weight) of Pembrolizumab in a single dose, or 200 mg of Pembrolizumab in a single dose, is administered at intervals of 3 weeks, or 400 mg of Pembrolizumab in a single dose is administered at intervals of 6 weeks.

[81] The agent according to [72] above, wherein 350 mg of Cemiplimab in a single dose is administered at intervals of 3 weeks.

[82] The agent according to [73] above, wherein 10 mg/kg (body weight) of Avelumab in a single dose is administered at intervals of 2 weeks.

[83] The agent according to [74] above, wherein 1200 mg of Atezolizumab in a single dose is administered at intervals of 3 weeks.

[84] The agent according to [75] above, wherein 10 mg/kg (body weight) of Durvalumab in a single dose is administered at intervals of 2 weeks, or 1500 mg of Durvalumab in a single dose is intravenously administered 4 times at intervals of 4 weeks.

[85] The agent according to [76] above, wherein 3 mg/kg (body weight) of Ipilimumab in a single dose, or 1 mg/kg (body weight) of Ipilimumab in a single dose, is intravenously administered 4 times at intervals of 3 weeks, or 1 mg/kg (body weight) of Ipilimumab in a single dose is administered at intervals of 6 weeks.

[86] The agent according to any one of [56] to [85] above, wherein the FOLFIRINOX therapy or its dose-reduced regimen is a therapy in which (2A) Oxaliplatin, (2B) Levofolinate calcium, (2C) Irinotecan hydrochloride hydrate, and (2D) Fluorouracil are administered in combination.

[87] The agent according to any one of [56] to [86] above, wherein the FOLFIRINOX therapy includes:

(2a) intravenously administering 50 to 85 mg/m$^2$ (body surface area) (preferably 50 mg/m$^2$ (body surface area), 65 mg/m$^2$ (body surface area), or 85 mg/m$^2$ (body surface area), among which 85 mg/m$^2$ (body surface area) is more preferable) of Oxaliplatin;
(2b) intravenously administering 200 mg/m$^2$ (body surface area) of Levofolinate calcium;
(2c) intravenously administering 90 to 180 mg/m$^2$ (body surface area) (preferably 90 mg/m$^2$ (body surface area), 120 mg/m$^2$ (body surface area), 150 mg/m$^2$ (body surface area), or 180 mg/m$^2$ (body surface area), among which 180 mg/m$^2$ (body surface area) is more preferable) of Irinotecan hydrochloride hydrate;
(2d) rapidly intravenously administering 400 mg/m$^2$ (body surface area) of Fluorouracil; and
(2e) after completion of the administration of Fluorouracil in (2d) above, further continuously intravenously administering 1200 to 2400 mg/m$^2$ (body surface area) (preferably 1200 mg/m$^2$ (body surface area), 1800 mg/m$^2$ (body surface area), or 2400 mg/m$^2$ (body surface area),
among which 2400 mg/m$^2$ (body surface area) is more preferable) of Fluorouracil.

[88] The agent according to any one of [56] to [86] above, wherein the dose-reduced regimen of the FOLFIRINOX therapy includes:

(2a) intravenously administering 50 to 85 mg/m$^2$ (body surface area) (preferably 50 mg/m$^2$ (body surface area), 65 mg/m$^2$ (body surface area), or 85 mg/m$^2$ (body surface area), among which 85 mg/m$^2$ (body surface area) is more preferable) of Oxaliplatin;
(2b) intravenously administering 200 mg/m$^2$ (body surface area) of Levofolinate calcium;
(2c) intravenously administering 120 to 150 mg/m$^2$ (body surface area) (preferably 150 mg/m$^2$ (body surface area)) of Irinotecan hydrochloride hydrate; and
(2e) continuously intravenously administering 1200 to 2400 mg/m$^2$ (body surface area) (preferably 1200 mg/m$^2$ (body surface area), 1800 mg/m$^2$ (body surface area), or 2400 mg/m$^2$ (body surface area), among which 2400 mg/m$^2$ (body surface area) is more preferable) of Fluorouracil.

[89] The agent according to any one of [56] to [88] above, wherein the FOLFIRINOX therapy or its dose-reduced regimen is a therapy wherein the administrations in series are performed at intervals of 2 to 4 weeks (preferably intervals of 2 weeks, intervals of 3 weeks, or intervals of 4 weeks, among which intervals of 2 weeks are more preferable).

[90] The agent according to any one of [56] to [87] and [89] above, wherein the FOLFIRINOX therapy includes:

(2a) intravenously administering 85 mg/m$^2$ (body surface area) of Oxaliplatin;
(2b) intravenously administering 200 mg/m$^2$ (body surface area) of Levofolinate calcium;
(2c) intravenously administering 180 mg/m$^2$ (body surface area) of Irinotecan hydrochloride hydrate;
(2d) rapidly intravenously administering 400 mg/m$^2$ (body surface area) of Fluorouracil; and
(2e) further continuously intravenously administering 2400 mg/m$^2$ (body surface area) of Fluorouracil after completion of the administration of Fluorouracil in (2d) above,

wherein the administrations in (2a), (2b), (2c), (2d), and (2e) above in series are performed at intervals of 2 weeks.

[91] The agent according to any one of [56] to [86], [88], and [89] above, wherein the dose-reduced regimen of the FOLFIRINOX therapy includes:

(2a) intravenously administering 85 mg/m$^2$ (body surface area) of Oxaliplatin;
(2b) intravenously administering 200 mg/m$^2$ (body surface area) of Levofolinate calcium;
(2c) intravenously administering 150 mg/m$^2$ (body surface area) of Irinotecan hydrochloride hydrate; and
(2e) continuously intravenously administering 2400 mg/m$^2$ (body surface area) of Fluorouracil,

wherein the administrations in (2a), (2b), (2c), and (2e) above in series are performed at intervals of 2 weeks.

[92] The agent according to any one of [56] to [87], [89], and [90] above, wherein the FOLFIRINOX therapy includes:

(2a) intravenously administering 85 mg/m$^2$ (body surface area) of Oxaliplatin over 2 hours;
(2b) intravenously administering 200 mg/m$^2$ (body surface area) of Levofolinate calcium over 2 hours (preferably after the completion of the administration of Oxaliplatin in (2a) above);
(2c) intravenously administering 180 mg/m$^2$ (body surface area) of Irinotecan hydrochloride hydrate over 1.5 hours from 30 minutes after start of the administration of Levofolinate calcium in (2b) above;
(2d) rapidly intravenously administering 400 mg/m$^2$ (body surface area) of Fluorouracil after completion of the administration of Levofolinate calcium in (2b) above; and
(2e) further continuously intravenously administering 2400 mg/m$^2$ (body surface area) of Fluorouracil over 46 hours after completion of the administration of Fluorouracil in (2d) above,

wherein a series of administrations in (2a), (2b), (2c), (2d), and (2e) above is performed at intervals of 2 weeks.

[93] The agent according to any one of [56] to [86], [88], [89], and [91] above, wherein the dose-reduced regimen of the FOLFIRINOX therapy includes:

(2a) intravenously administering 85 mg/m$^2$ (body surface area) of Oxaliplatin over 2 hours;
(2b) intravenously administering 200 mg/m$^2$ (body surface area) of Levofolinate calcium over 2 hours (preferably after the completion of the administration of Oxaliplatin in (2a) above);
(2c) intravenously administering 150 mg/m$^2$ (body surface area) of Irinotecan hydrochloride hydrate over 1.5 hours from 30 minutes after start of the administration of Levofolinate calcium in (2b) above; and
(2e) continuously intravenously administering 2400 mg/m$^2$ (body surface area) of Fluorouracil over 46 hours after completion of the administration of Levofolinate calcium in (2b) above,

wherein a series of administrations in (2a), (2b), (2c), and (2e) above is performed at intervals of 2 weeks.

[94] The agent according to any one of [56] to [93] above, wherein the FOLFIRINOX therapy or its dose-reduced regimen, and the administration of the immune checkpoint inhibitory substance and the CD47 inhibitory substance, are started on the same day.

[95] An agent for suppressing progression of, suppressing recurrence of, and/or treating a cancer, comprising a CD47 inhibitory substance as an active ingredient, the agent being administered in combination with FOLFIRINOX therapy or its dose-reduced regimen, and with an immune checkpoint inhibitory substance, wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,
(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and
(iii) the FOLFIRINOX therapy includes:

(2a) intravenously administering 85 mg/m$^2$ (body surface area) of Oxaliplatin (preferably over 2 hours);
(2b) intravenously administering 200 mg/m$^2$ (body surface area) of Levofolinate calcium (preferably over 2 hours) (preferably after the completion of the administration of Oxaliplatin in (2a) above);
(2c) intravenously administering 180 mg/m$^2$ (body surface area) of Irinotecan hydrochloride hydrate (preferably over 1.5 hours) (preferably from 30 minutes after start of the administration of Levofolinate calcium in (2b) above);
(2d) rapidly intravenously administering 400 mg/m$^2$ (body surface area) of Fluorouracil after completion of the administration of Levofolinate calcium in (2b) above; and
(2e) further continuously intravenously administering 2400 mg/m$^2$ (body surface area) of Fluorouracil (preferably over 46 hours) after completion of the administration of Fluorouracil in (2d) above,
wherein the series of administrations in (2a), (2b), (2c), (2d), and (2e) above is performed at intervals of 2 weeks, or

the dose-reduced regimen of the FOLFIRINOX therapy includes:

(2a) intravenously administering 85 mg/m$^2$ (body surface area) of Oxaliplatin (preferably over 2 hours);
(2b) intravenously administering 200 mg/m$^2$ (body surface area) of Levofolinate calcium (preferably over 2 hours) (preferably after the completion of the administration of Oxaliplatin in (2a) above);
(2c) intravenously administering 150 mg/m$^2$ (body surface area) of Irinotecan hydrochloride hydrate (preferably over 1.5 hours) (preferably from 30 minutes after start of the administration of Levofolinate calcium in (2b) above); and
(2e) continuously intravenously administering 2400 mg/m$^2$ (body surface area) of Fluorouracil (preferably over 46 hours) after completion of the administration of Levofolinate calcium in (2b) above,
wherein a series of administrations in (2a), (2b), (2c), and (2e) above is performed at intervals of 2 weeks.

[96] An agent for suppressing progression of, suppressing recurrence of, and/or treating a cancer, comprising an immune checkpoint inhibitory substance as an active ingredient, the agent being administered in combination with FOLFIRINOX therapy or its dose-reduced regimen, and with a CD47 inhibitory substance, wherein

(i) the CD47 inhibitory substance is Magrolimab, and 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations,
(ii) the immune checkpoint inhibitory substance is Nivolumab, and 240 mg of Nivolumab in a single dose is administered at intervals of 2 weeks, 360 mg of the same in a single dose is administered at intervals of 3 weeks, or 480 mg of the same in a single dose is administered at intervals of 4 weeks (preferably, 480 mg of the same in a single dose is administered at intervals of 4 weeks), and
(iii) the FOLFIRINOX therapy includes:

(2a) intravenously administering 85 mg/m$^2$ (body surface area) of Oxaliplatin (preferably over 2 hours);
(2b) intravenously administering 200 mg/m$^2$ (body surface area) of Levofolinate calcium (preferably over 2 hours) (preferably after the completion of the administration of Oxaliplatin in (2a) above);
(2c) intravenously administering 180 mg/m$^2$ (body surface area) of Irinotecan hydrochloride hydrate (preferably over 1.5 hours) (preferably from 30 minutes after start of the administration of Levofolinate calcium in (2b) above);
(2d) rapidly intravenously administering 400 mg/m$^2$ (body surface area) of Fluorouracil after completion of the administration of Levofolinate calcium in (2b) above; and
(2e) further continuously intravenously administering 2400 mg/m$^2$ (body surface area) of Fluorouracil (preferably over 46 hours) after completion of the administration of Fluorouracil in (2d) above,
wherein the series of administrations in (2a), (2b), (2c), (2d), and (2e) above is performed at intervals of 2 weeks, or

the dose-reduced regimen of the FOLFIRINOX therapy includes:

(2a) intravenously administering 85 mg/m$^2$ (body surface area) of Oxaliplatin (preferably over 2 hours);

(2b) intravenously administering 200 mg/m$^2$ (body surface area) of Levofolinate calcium (preferably over 2 hours) (preferably after the completion of the administration of Oxaliplatin in (2a) above);

(2c) intravenously administering 150 mg/m$^2$ (body surface area) of Irinotecan hydrochloride hydrate (preferably over 1.5 hours) (preferably from 30 minutes after start of the administration of Levofolinate calcium in (2b) above); and

(2e) continuously intravenously administering 2400 mg/m$^2$ (body surface area) of Fluorouracil (preferably over 46 hours) after completion of the administration of Levofolinate calcium in (2b) above,

wherein a series of administrations in (2a), (2b), (2c), and (2e) above is performed at intervals of 2 weeks.

[97] The agent according to [95] or [96] above, wherein the cancer is pancreatic cancer having distant metastasis.

[98] The agent according to [97] above, wherein the agent is administered as a primary therapy to a patient who has not been treated with a systemic anti-malignant tumor agent against pancreatic cancer having distant metastasis.

[99] The agent according to any one of [95] to [98] above, wherein the FOLFIRINOX therapy or its dose-reduced regimen, and the administration of Magrolimab and Nivolumab, are started on the same day.

Unless defined otherwise, all technical, scientific terms, and abbreviations used herein have the same meaning as commonly understood by one of ordinary skill in the art to which this invention belongs.

In addition, in the present specification, all contents of all patent documents and non-patent documents or references explicitly cited may be cited herein as part of the present specification.

EXAMPLES

Hereinafter, the present invention will be described in detail with reference to Examples, but the present invention is not limited thereto.

Example 1: Open-label, uncontrolled study that uses, as a primary therapy, Magrolimab and Nivolumab in combination with a combination therapy in which Bevacizumab or Cetuximab is added to FOLFOX therapy as a standard therapy, to patients with radically unresectable advanced or recurrent colorectal cancer

An object of the present test is to study tolerability, safety, and efficacy when Magrolimab and Nivolumab, and a combination therapy in which Bevacizumab or Cetuximab is added to FOLFOX therapy, are used in combination as a primary therapy, to patients with radically unresectable advanced or recurrent colorectal cancer. This clinical trial can evaluate the effect when Magrolimab, Nivolumab, and the combination therapy in which Bevacizumab or Cetuximab is added to FOLFOX therapy, are used in combination.

(1) Object patient

Patients with radically unresectable advanced or recurrent colorectal cancer

(2) Patient selection criteria

Patients meeting all of the following criteria at the time of registration were selected. When it was found that the following criteria were not satisfied before the first administration of the investigational drug, the administration of the study drug was not started and the clinical trial was stopped.

1. Gender: any.
2. Age (when giving consent): 20 years old or more.
3. A patient who can be hospitalized from the day before the start of investigational drug administration until at least the end of the test on Day 15 after the start of investigational drug administration (tolerability evaluation part only).
4. A patient who is pathologically diagnosed with adenocarcinoma of colon or rectum origin histologically (excluding appendix cancer and anal tube cancer) and for whom radical resection is not indicated.
5. A patient with measurable lesion as defined in RECIST Guideline Version 1.1 in image diagnosis within 28 days prior to administration of the investigational drug.

- A patient, having received radiation therapy for a measurable lesion, with progression being confirmed in image diagnosis performed after the radiation therapy.

6. A patient who has not been treated with a systemic anti-malignant tumor agent for radically unresectable advanced or recurrent colorectal cancer.

- No recurrence has been observed within 6 months after completion of adjuvant therapy when preoperative/postoperative adjuvant therapy is performed in combination with curative surgery (R0 resection has been confirmed).

7. A patient capable of providing tumor tissue specimens for biomarker testing.

- Tumor tissue specimens are acquired during the screening period. When a new tumor biopsy is difficult, a preserved specimen can be used. However, the preserved specimen has been collected within 180 days before administration of the investigational drug and after the last administration of the most recent systemic anticancer therapy.

8. A RAS mutant type patient scheduled to receive the combination therapy of FOLFOX (Fluorouracil, Levofolinate, Oxaliplatin) and Bevacizumab (Cohort 1), or a RAS wild-type patient that is scheduled to receive the combination therapy of FOLFOX (Fluorouracil, Levofolinate, Oxaliplatin) and Cetuximab and has a primary tumor site on the left side (descending colon, sigmoid colon, rectum) (Cohort 2).

9. A patient with ECOG Performance Status of 0 to 1.

10. A patient expected to survive for 3 months or more at registration.

11. For a woman who may become pregnant (including a patient who does not have menstruation due to medical reasons such as chemical menopause), a patient who has agreed to perform double contraception for a period since giving consent up to 5 months after the last administration of the investigational drug and for the contraceptive period specified by each drug after the last administration of the standard therapeutic drug. In addition, a patient agreed not to breast feed for a period since giving consent up to 5 months after the last administration of the investigational drug and for a period during which lactation is restricted by each drug after the last administration of the standard therapeutic agent. Here, a woman who may become pregnant encompasses all women who have experienced menses, have not undergone sterilization (hysterectomy, bilateral tubal ligation, or bilateral oophorectomy, etc.), and have not undergone menopause. The definition of menopause is amenorrhea for 12 consecutive months or more despite no reason to mention. A woman using oral contraceptive or an intrauterine device or a pessary is considered to be likely to become pregnant.

12. In the case of a male, a patient who has agreed to perform double contraception for a period since the start of the administration of the investigational drug up to 4 months after the last administration of the investigational drug and for the contraceptive period specified for each drug after the last administration of the standard therapeutic agent. Here, regarding contraception, it is necessary to perform double contraception by any two of vasectomy or condom of a male patient or a male partner, fallopian tube ligation of a female patient or a female partner, a pessary, an intrauterine device, or an oral contraceptive.

13. A patient whose latest laboratory test values obtained within 14 days before the start of the administration of the investigational drug meet the following criteria. The patient should not receive administration of a hematopoietic factor preparation such as granulocyte colony stimulating factor (G-CSF) or blood transfusion within 14 days before the test.

- The neutrophil count is 1,500/mm$^3$ or more.
- The number of platelets is 100,000/mm$^3$ or more.
- Hemoglobin is 11.0 g/dL or more.
- AST (GOT) and ALT (GPT) are 3.0 times or less the upper limit of the facility reference value (however, with liver metastasis, AST (GOT) and ALT (GPT) are 5.0 times or less the upper limit of the facility reference value).
- Total bilirubin is 1.5 times or less the upper limit of the facility reference value (however, in the case of a patient with Gilbert's syndrome, total bilirubin is 3.0 times or less the upper limit of the facility reference value.).
- Serum creatinine is 1.5 times or less the upper limit of the facility reference value, or creatinine clearance (measured value or estimated value by Cockcroft/Gault equation) is 40 mL/min or more.
- Albumin is 3.0 g/dL or more.
- [Cohort 1 only] Urine protein is 1+ or less. However, a patient with urine protein of 2+ or more in a urine test or a urine test paper test can be registered when the urine protein is 1 g/24 hours or less in a 24 hour urine collection, or when the urine protein/creatinine ratio (UPCR) is less than 1 g/gCr.

14. A patient who has been sufficiently informed of the contents of the present clinical trial by the principal investigator or the co-investigator using the written consent and the written description and who freely agrees to participate in the present clinical trial.

(3) Patient exclusion criteria

Patients meeting any of the following criteria at the time of registration were excluded. When it was found that the patient met any of the following criteria before the first administration of the investigational drug, the administration of the investigational drug was not started and the clinical trial was stopped.

1. A RAS/BRAF wild type patient with a primary tumor site on the right (cecum, ascending colon, transverse colon).
2. A BRAF mutated-type patient and a patient with frequent microsatellite instability (MSI-High) or mismatch repair mechanism deficiency (dMMR).
3. A patient subject to anticoagulation therapy, or with a disease in need of anticoagulation therapy.
4. A patient with persistent gastrointestinal bleeding ≥ Grade 2.
5. A patient with complicated gastrointestinal perforation, severe fistula, or tracheoesophageal fistula.
6. A patient with accumulation of pericardial fluid, pleural effusion or ascites which cannot be managed by pharmacotherapy.
7. A patient with a history of advanced hypersensitivity reactions to antibody formulations.
8. A patient with contraindications to administration of any drug in the standard therapy used in each cohort.

[Cohort 1] Fluorouracil, Levofolinate, Oxaliplatin, and Bevacizumab.
[Cohort 2] Fluorouracil, Levofolinate, Oxaliplatin, and Cetuximab.

9. Patients with Grade ≥ 2 peripheral neuropathy.
10. A patient with complication of autoimmune disease or history of chronic or recurrent autoimmune disease. However, a patient complicated with a skin disease that does not require systemic therapy (vitiligo, psoriasis, alopecia, etc.) or a disease that is not considered to recur in the absence of an external trigger can be registered.
11. A patient who had undergone surgical therapy that was determined by the principal investigator or co-investigator to have an effect on the safety and efficacy evaluation of the investigational drug.
12. A patient with double cancer. However, a patient with completely resected basal cell carcinoma, stage I squamous cell carcinoma, carcinoma in situ, intramucosal carcinoma or superficial bladder cancer, or another cancer that has not recurred for 3 years or more can be enrolled.
13. A patient with a metastatic lesion in the brain or meninges. However, a patient who is asymptomatic and does not require treatment can be registered.
14. A patient with interstitial lung disease diagnosed by diagnostic imaging or clinical findings, or complication or history of pulmonary fibrosis.
15. A patient whose tumor-associated pain is not stably controlled.
16. A patient with a history of hemolytic anemia within 90 days prior to registration.
17. A patient who has a history of transient ischemic attack, cerebrovascular accident, thrombosis or thromboembolism (pulmonary embolism or deep vein thrombosis) within 180 days before enrollment and has poor control of thrombosis or thromboembolism. However, central venous catheter-related venous thrombi are tolerated.
18. A patient with the following uncontrolled or significant cardiovascular disease.

- Acute myocardial infarction within 6 months prior to enrollment.
- Unstable angina or angina requiring treatment.
- New York Heart Association (NYHA) grade III to IV congestive heart failure.
- Uncontrolled hypertension (systolic blood pressure of 150 mmHg or more or diastolic blood pressure of 100 mmHg or more is maintained for 24 hours or more.) despite appropriate treatment.
- Uncontrolled or significant arrhythmia.

19. A patient complicated with uncontrolled diabetes.
20. A patient with a systemic infection in need of treatment.
21. A patient in need of or having a history of transplant therapy (except autologous transplants).
22. A patient who is positive for any of the HIV-1 antibody and HIV-2 antibody tests, the HTLV-1 antibody test, the HBs antigen test, and the HCV antibody test. In addition, a patient who has a negative HBs antigen test but is positive for either an HBs antibody test or an HBc antibody test and has HBV-DNA quantification of not less than detection sensitivity. However, if the HCV antibody test is positive but HCV-RNA is negative, registration is allowed.
23. A patient who received the following treatments prior to giving consent and prior to administration of the investigational drug.

- A patient who has previously been treated with a drug targeting the Magrolimab, CD47-SIRPα pathway.
- A patient who has previously had a treatment history including an antibody therapy for the purpose of T-cell control using anti-PD-1 antibody, anti-PD-L1 antibody, anti-PD-L2 antibody, anti-CTLA-4 antibody, or the like, or a cancer vaccine.
- A patient who received more than 2 units of red blood cell transfusion within 28 days prior to consent acquisition.
- A patient who received surgical therapy with local anesthesia within 14 days prior to investigational drug administration.
- A patient who underwent adhesion surgery such as pleurodesis or pericardiodesis within 14 days prior to investigational drug administration.
- A patient who received administration of a >10 mg daily prednisone-equivalent systemic adrenocortical hormone (except for temporary use for the purpose of examination, local administration, treatment and prevention of allergic reaction of contrast medium, or reduction of edema associated with radiotherapy, or the like) or immunosuppressive agent within 14 days prior to investigational drug administration.
- A patient who received palliative local radiation therapy within 28 days prior to investigational drug administration.
- A patient who received surgical therapy with general anesthesia within 28 days prior to investigational drug administration.
- A patient who received live or attenuated vaccination within 28 days prior to investigational drug administration.
- A patient who received a radiopharmaceutical (except for the use of a radiopharmaceutical for testing and diagnosis) within 56 days prior to investigational drug administration.
- A patient who received other unapproved drugs (including administration by clinical studies, unapproved combination drugs, and new dosage forms) within 28 days prior to investigational drug administration.

24. A patient who may be pregnant, lactating or pregnant.
25. A patient who is determined to be in a condition lacking consent ability due to dementia complication or the like.
26. In addition, a patient judged by the principal investigator or the co-investigator to be unsuitable as a research subject of the present clinical trial.

(4) Dosage and duration of administration

[Magrolimab]

On Day 1 of the first cycle, 1 mg/kg of Magrolimab was intravenously administered over 3 hours (± 30 minutes) as an initial administration, and then 20 mg/kg or 30 mg/kg of the same in a single does was intravenously administered over 2 hours (± 30 minutes) at intervals of 1 week in the first cycle. In the second and subsequent cycles, 20 mg/kg or 30 mg/kg of Magrolimab in a single dose was intravenously administered over 2 hours (±30 minutes) at intervals of 2 weeks, and the administration was continued until predetermined administration stopping criteria for Magrolimab were met. When Magrolimab, Nivolumab, and a combination therapy in which Bevacizumab or Cetuximab was added to FOLFOX therapy were administered on the same day, the administration was started in the order of Magrolimab, Nivolumab, and the combination therapy in which Bevacizumab or Cetuximab was added to FOLFOX therapy.

[Nivolumab]

Nivolumab, 480 mg, was intravenously administered over 30 minutes at intervals of 4 weeks, and was continuously administered until predetermined administration stopping criteria for Nivolumab were met. The administration of Nivolumab was performed on and after Day 25, with an interval of at least 24 days from the previous administration.

[Combination therapy in which Bevacizumab or Cetuximab is added to FOLFOX therapy]

For the selection of Bevacizumab or Cetuximab, Bevacizumab was used for RAS mutant type patients, and Cetuximab was used for RAS wild-type patients.
The starting dose of Bevacizumab was 5 mg/kg (body weight), and the Bevacizumab was administered by intravenous infusion over 90 minutes. If the first dose was well tolerated, the second dose could be administered over 60 minutes. If the second dose was also well tolerated, the subsequent dose could be administered over 30 minutes. The administration interval was 2 weeks or longer.
The starting dose of Cetuximab was 400 mg/m$^2$ (body surface area), and the Cetuximab was administered by intravenous infusion over 2 hours. As the second and subsequent doses, 250 mg/m$^2$ was administered by intravenous infusion over 1 hour once a week or at intervals of 2 weeks. Alternatively, if necessary, Cetuximab of 500 mg/m$^2$ could be administered by intravenous infusion over 2 hours once a two weeks, i.e., at intervals of 2 weeks. The dose was appropriately reduced according to the condition of the patient.

(1x) After administration of Bevacizumab or Cetuximab, (1a) 85 mg/m$^2$ (body surface area) of oxaliplatin was intravenously administered over 2 hours. (1b) 200 mg/m$^2$ (body surface area) of Levofolinate calcium was intravenously administered over 2 hours, simultaneously with the administration of Oxaliplatin. (1c) 400 mg/m$^2$ (body surface area) of Fluorouracil was rapidly intravenously administered after the completion of intravenous administration of Levofolinate calcium, and (1d) 2400 mg/m$^2$ (body surface area) of the same was then administered intravenously over 46 hours. The series of administrations in (1x), (1a), (1b), (1c), and (1d) above was performed at intervals of 2 weeks. Commercially available products were used for Bevacizumab, Cetuximab, Oxaliplatin, Levofolinate calcium, and Fluorouracil.

[Clinical trial schedule]

The present test consists of a screening period, a treatment period, and an observation period. The summary of the clinical trial schedule is shown in Fig. 1.

The screening period was within 28 days prior to investigational drug administration, and the principal investigator or co-investigator included, in the clinical trial, patients who met the above selection criteria, did not meet the above exclusion criteria, and were determined to be eligible for the clinical trial.

The treatment period was set to 28 days per cycle, and the first day of administration of the investigational drug was set to Day 1 of Cycle 1. The first day of each cycle after the second cycle was Day [28 × (the number of cycles -1) + 1]. The administration of Magrolimab, Nivolumab, and a combination therapy in which Bevacizumab or Cetuximab was added to FOLFOX therapy was started according to each dosage and administration, and the administration was continued according to respective administration criteria, dose-reduction criteria, and doses upon reduction for Magrolimab, Nivolumab, and the combination therapy in which Bevacizumab or Cetuximab was added to FOLFOX therapy. The time point at which the evaluation at the completion (stop) of administration of Magrolimab, Nivolumab, and the combination therapy in which bevacizumab or cetuximab was added to FOLFOX therapy was completed was defined as the end of the treatment period. Among all research subjects who received the investigational drug, research subjects to whom the administration of Magrolimab, Nivolumab, and the combination therapy in which Bevacizumab or Cetuximab was added to FOLFOX therapy was stopped or completed underwent evaluation at the completion (stop) of administration and entered the observation period. Follow-up was performed after the completion of the observation period.

[Administration criteria for Magrolimab and Nivolumab]

At the start of each administration, research subjects must meet all predetermined administration criteria determined in view of the administration criteria in the clinical trials carried out to date regarding Magrolimab and Nivolumab. If any of these criteria were not met, the scheduled Magrolimab and Nivolumab administrations were withdrawn. The test was performed once a week or more frequently as clinically necessary for the research subject who had stopped taking the drug, to determine the possibility of restarting the administration.

[Administration stopping criteria for Magrolimab]

In the treatment period, the administration of Magrolimab was stopped for a research subject who met any of predetermined administration stopping criteria determined in view of the administration stopping criteria in the clinical trials carried out to date regarding Magrolimab.

[Administration stopping criteria for Nivolumab]

In the treatment period, the administration of Nivolumab was stopped for a research subject who met any of predetermined administration stopping criteria determined in view of the administration stopping criteria in the clinical trials carried out to date regarding Nivolumab.

[Administration criteria for combination therapy in which Bevacizumab or Cetuximab is added to FOLFOX therapy]

At the start of each administration, a research subject must meet all predetermined administration criteria determined in view of the latest package insert for combination therapy in which Bevacizumab or Cetuximab is added to FOLFOX therapy. The administration was postponed until the laboratory test values on the administration scheduled date recovered to states satisfying the conditions of the standard, and the administration was performed after confirming that it did not correspond to the contraindication of each drug.

[Dose reduction and dose reduction criteria for combination therapy in which Bevacizumab or Cetuximab is added to FOLFOX therapy]

Dose reduction was performed according to the latest package insert.

[Stopping criteria for combination therapy in which Bevacizumab or Cetuximab is added to FOLFOX therapy]

In the treatment period, for research subjects who met any of the predetermined administration stopping criteria determined in view of the latest package insert regarding the combination therapy in which Bevacizumab or Cetuximab was added to FOLFOX therapy, the administration of the combination therapy in which bevacizumab or cetuximab was added to FOLFOX therapy was stopped.

[Evaluation criteria of efficacy]

(Image diagnosis)

CT/nuclear magnetic resonance imaging (MRI) imaging and the like of the chest, abdomen and pelvis were performed.
The principal investigator or co-investigator measured the tumor diameter of the target lesion according to RECIST Guideline Version 1.1, and determined the antitumor effect.

(Evaluation items)

(1) Response rate (ORR), (2) disease control rate (DCR), (3) overall survival (OS), (4) progression-free survival (PFS), (5) duration of response (DOR), (6) time to response (TTR), (7) best overall response (BOR), (8) rate of change in sum of tumor diameters of target lesions, (9) maximum rate of change in sum of tumor diameters of target lesions, (10) changes in tumor markers (CEA and CA19 -9)

(Evaluation of target lesion)

Complete response (CR)

It means the disappearance of all non-lymph node target lesions and the reduction of the minor axis of all lymph node lesions selected as target lesions to less than 10 mm.

Partial response (PR)

This means that the sum of diameters of the target lesion decreases by 30% or more as compared with the baseline sum of diameters.

Progressive disease (PD)

This means a case where the sum of diameters of the target lesion is increased by 20% or more as compared with the minimum sum of diameters during the course, and the sum of diameters is increased by 5 mm or more as an absolute value.

Stable disease (SD)

This means a case where there is no reduction corresponding to PR and there is no increase corresponding to PD as compared with the minimum sum of diameters during the course.

Not Evaluable (NE)

This means a case where the test cannot be performed for some reason or a case where it cannot be determined as any of CR, PR, PD, and SD.

(1) Response rate

Response rate indicates the percentage of research subjects whose best overall response was determined to be CR or PR.

(2) Disease control rate

Disease control rate indicates the percentage of research subjects whose best overall response was determined to be CR, PR, or SD.

(3) Overall survival

The overall survival is calculated from the following equation.

$$\text{Overall survival (days)} = \text{“date of death due to any cause”} - \text{“date of starting administration of investigational drug”} + 1$$

Note that, for a research subject who became untraceable or who did not die by the data cut-off date, the final survival confirmation date is defined as the termination date.

(4) Progression-free survival

The progression-free survival is calculated from the following formula.

$$\text{Progression-free survival (days)} = \text{“earlier of date when overall effect was determined as PD and date of death due to any cause”} - \text{“date of starting administration of investigational drug”} + 1$$

Note that, for a research subject whose total effect has not been determined to be PD and who has not died, the date on which the last evaluable image diagnosis was performed is defined as the termination date. For a research subject who has not undergone evaluable diagnostic imaging and who has not died, the date of starting administration of investigational drug is defined as the termination date. For a research subject who received post-treatment for cancer before the overall effect was determined to be PD or died, the day on which the last evaluable imaging diagnosis before the start of post-treatment for cancer was performed is defined as the termination date.

(5) Duration of response

The duration of response is calculated from the following equation.

$$\text{Duration of response (days)} = \text{“earlier of date when total response was determined to be PD for first time after confirmation of response, and date of death due to any cause”} - \text{“date of first determination of confirmed CR or PR”} + 1$$

The subject to be evaluated is a research subject who shows CR or PR determined through the clinical trial.

(6) Time to response

The time to response is calculated from the following equation.

$$\text{Time to response (days)} = \text{``date of first determination of confirmed CR or PR''} -$$

$$\text{``date of starting administration of investigational drug''} + 1$$

(7) Best overall response (BOR)

The best overall response is determined from the overall response determined by the completion of the clinical trial. However, CR and PR require confirmation by continuous evaluation after an interval of 4 weeks (28 days) or more, and are determined according to the criteria in Table 1.

[Table 1]

First overall evaluation
Subsequent overall evaluation
Best overall response
SD, PD, or PR
SD when satisfying minimum reference for SD, and PD in the other cases
SD when satisfying minimum reference for SD, and NE in the other cases

(8) Rate of change in sum of tumor diameters of target lesions

For a research subject having a target lesion, the rate of change in sum of tumor diameters of target lesions is calculated using the following calculation formula. However, the rate of change in sum of tumor diameters of target lesions after the post-treatment is performed is not calculated.

[Mathematical formula 1]

$$\text{Rate of change (\%)} = (\text{``sum of tumor diameters in each of evaluation time points} -$$

$$\text{sum of tumor diameters before administration''}) / (\text{``sum of tumor diameters before}$$

$$\text{administration''}) \times 100$$

(9) Maximum rate of change in sum of tumor diameters of target lesions

The maximum rate of change in sum of tumor diameters of target lesions is defined as a rate of change at the time point when the sum of tumor diameters of the target lesions is the smallest. However, the sum of the tumor diameters of the target lesions after the total effect is determined to be PD or after the post-treatment is performed is not used for calculating the maximum rate of change.

[Mathematical formula 2]

$$\text{Maximum rate of change (\%)} = (\text{``smallest sum of tumor diameters after}$$

$$\text{administration - sum of tumor diameters before administration''}) / (\text{``sum of tumor diameters}$$

$$\text{before administration''}) \times 100$$

(10) Change in rate of change in tumor marker (CEA and CA19-9)

The rate of change in the tumor marker is calculated using the following calculation formula. However, the rate of change in the tumor marker after the post-treatment is performed is not calculated.

[Mathematical formula 3]

$$\text{Rate of change (\%)} = (\text{"tumor marker in each of evaluation time points - tumor marker before administration"}) / (\text{"tumor marker before administration"}) \times 100$$

[Evaluation items of safety]

The following items were measured, examined and investigated by the principal investigator and the like at a predetermined time.
(1) Dose-limiting toxicity (DLT), (2) adverse events, (3) laboratory tests (hematological tests, blood biochemical tests, immunological tests, hormonal tests, blood coagulation system tests, urine qualitative tests), peripheral blood smear, (4) vital signs (systolic/diastolic blood pressure, pulse rate, body temperature, respiratory rate), transcutaneous oxygen saturation ($SpO_2$), body weight, (5) 12-lead electrocardiogram, (6) ECOG Performance Status, and (7) chest x-ray

[Results of efficacy evaluation]

For Magrolimab, Nivolumab, and the combination therapy in which Bevacizumab was added to FOLFOX therapy, there were two cases determined to be PR at the time point of 8 months after the registration of the first research subject.
For Magrolimab, Nivolumab, and the combination therapy in which Cetuximab was added to FOLFOX therapy, there were three cases determined to be PR at the time point of 8 months after the registration of the first research subject.

Example 2: Open-label, uncontrolled study that uses, as a primary therapy, Magrolimab, Nivolumab in combination with mFFX therapy as standard therapy, to patients with pancreatic cancer having distant metastasis

An object of the present test is to study tolerability, safety, and efficacy when Magrolimab, Nivolumab, and mFFX therapy are used in combination as a primary therapy, to pancreatic cancer patients having distant metastasis. This clinical trial can evaluate the effect when Magrolimab, Nivolumab, and the mFFX therapy are used in combination.

(1) Object patient

Patients with pancreatic cancer having distant metastasis

(2) Patient selection criteria

Patients meeting all of the following criteria at the time of registration were selected. When it was found that the following criteria were not satisfied in a period from the registration to the first administration of the investigational drug, the administration of the study drug was not started and the clinical trial was stopped.

1. Gender: any.
2. Age (when giving consent): 20 years old or more.
3. A patient who can be hospitalized from the day before the start of investigational drug administration until at least the end of the test on Day 15 after the start of investigational drug administration (tolerability evaluation part only).
4. A patient with invasive pancreatic ductal cancer diagnosed as adenocarcinoma by tissue or cytology.
5. A patient who has not been treated with a systemic anti-malignant tumor agent for pancreatic cancer having distant metastasis. However, when a patient who has received chemoradiotherapy, postoperative adjuvant chemotherapy, or preoperative adjuvant chemotherapy has relapsed 180 days or more after the end of administration of the antineoplastic agent, registration is allowed.
6. A patient with measurable lesion as defined in RECIST Guideline Version 1.1 in image diagnosis within 14 days prior to administration of the investigational drug. However, in the case of having only a measurable lesion with a history of radiation therapy, the lesion is limited to a lesion confirmed to have exacerbation in diagnostic imaging after radiation therapy is administered.
7. A patient with Eastern Cooperative Oncology Group (ECOG) Performance Status of 0 or 1.
8. A patient expected to survive for 90 days or more.
9. A patient capable of providing tumor tissue specimens for biomarker testing.
Tumor tissue specimens are acquired during the screening period. When a new tumor biopsy is difficult, a preserved specimen can be used. However, the preserved sample is collected within 180 days before investigational drug administration.

10. Patient, when being a woman who may become pregnant (including patient who do not have menstruation due to medical reasons such as chemical menopause), who, since giving consent, has agreed to perform double contraception for 5 months after the last administration of the investigational drug and for the period for which contraception is prescribed for each drug after the last administration of the standard therapeutic drug. In addition, a patient agreed not to breast feed for a period since giving consent up to 5 months after the last administration of the investigational drug and for a period during which lactation is restricted by each drug after the last administration of the standard therapeutic agent. Here, a woman who may become pregnant encompasses all women who have experienced menses, have not undergone sterilization (hysterectomy, bilateral tubal ligation, or bilateral oophorectomy, etc.), and have not undergone menopause. The definition of menopause is amenorrhea for 12 consecutive months or more despite no reason to mention. A woman using oral contraceptive or an intrauterine device or a contraceptive pessary is considered to be likely to become pregnant.

11. In the case of a male, a patient who has agreed to perform double contraception for a period since the start of the administration of the investigational drug up to 4 months after the last administration of the investigational drug and for the period for which contraception is prescribed for each drug after the last administration of the standard therapeutic agent. Here, regarding contraception, it is necessary to perform double contraception by any two of vasectomy or condom of a male patient or a male partner, fallopian tube ligation of a female patient or a female partner, a contraceptive pessary, an intrauterine device, or an oral contraceptive.

12. A patient whose latest laboratory test values obtained within 14 days before the start of the administration of the investigational drug meet the following criteria. The patient should not receive administration of a hematopoietic factor preparation such as granulocyte colony stimulating factor (G-CSF formulation) or blood transfusion within 14 days before the test day.

- Neutrophil count: 2,000/mm$^3$ or more.
- The number of platelets: 100,000/mm$^3$ or more.
- Hemoglobin: 11.0 g/dL or more.
- AST (GOT) and ALT (GPT): 3.0 times or less the upper limit of the facility reference value (however, with liver metastasis, 5.0 times or less the upper limit of the facility reference value).
- Total bilirubin: 1.5 times or less the upper limit of the facility reference value.
- Albumin: 3.0 g/dL or more.
- Creatinine: 1.5 times or less the upper limit of the facility reference value, or creatinine clearance (measured value or estimated value by Cockcroft/Gault equation) is 40 mL/min or more.

13. A patient who has been sufficiently informed of the contents of the present clinical trial by the principal investigator or the co-investigator using the written consent and the written description and who freely agrees to participate in the present clinical trial.

(3) Patient exclusion criteria

Patients who were considered to meet any of the following criteria at the time of registration were excluded. When it was found that the patient met any of the following criteria in a period from the enrollment to the first administration of the investigational drug, the administration of the investigational drug was not started and the clinical trial was stopped.

1. A patient for whom the administration of Oxaliplatin, Irinotecan, Fluorouracil or Levofolinate calcium is contraindicated.
2. A patient with clinically significant diarrhea (including watery feces).
3. A patient with peripheral motor neuropathy or peripheral sensory neuropathy.
4. A patient with UDP glucuronic acid transferase 1A1(UGT1A1) gene polymorphism homozygote (UGT1A1*6/*6, UGT1A1*28/*28) or heterozygote (complex heterozygote: UGT1A1*6/*28).
5. A patient with complication or history of advanced hypersensitivity reactions to antibody formulations.
6. A patient who had undergone surgical therapy that was determined by the principal investigator or co-investigator to have an effect on the efficacy and safety evaluation of the investigational drug.
7. A patient with complication of autoimmune disease or history of chronic or recurrent autoimmune disease. However, a patient complicated with Type 1 diabetes, hypothyroidism manageable with hormone replacement therapy, or with a skin disease that does not require systemic therapy (vitiligo, psoriasis, alopecia, etc.) can be registered.
8. A patient with multiple cancers (a patient with completely resected basal cell carcinoma, stage I squamous cell carcinoma, carcinoma in situ, intramucosal carcinoma or superficial bladder cancer, or another cancer that has not recurred for 3 years or more can be enrolled.

9. A patient with a metastatic lesion in the brain or meninges. However, a patient who is asymptomatic and does not require treatment can be registered.

10. A patient with interstitial lung disease diagnosed by diagnostic imaging or clinical findings, or complication or history of pulmonary fibrosis.

11. A patient complicated with diverticulitis or symptomatic gastrointestinal ulcer disease.

12. A patient with accumulation of pericardial fluid, pleural effusion or ascites which cannot be managed by pharmacotherapy.

13. A patient complicated with unmanageable cancer pain.

14. A patient with high peritoneal dissemination, such as continuous accumulation of ascites beyond the pelvic cavity.

15. A patient in need of or having a history of transplant therapy (except autologous transplants).

16. A patient with a history of hemolytic anemia within 90 days prior to registration.

17. A patient who has a history of transient ischemic attack, cerebrovascular accident, thrombosis or thromboembolism (pulmonary embolism or deep vein thrombosis) within 180 days before registration.

18. A patient with the following uncontrolled or significant cardiovascular disease.

- Myocardial infarction within 180 days prior to registration.
- Uncontrolled angina within 180 days prior to registration.
- New York Heart Association (NYHA) grade III or IV congestive heart failure.
- Uncontrolled hypertension (systolic blood pressure of 150 mmHg or more or diastolic blood pressure of 90 mmHg or more is maintained for 24 hours or more.) despite appropriate treatment.
- Arrhythmia requiring treatment.

19. A patient complicated with unmanageable diabetes.

20. A patient with a systemic infection in need of treatment.

21. A patient who is positive for any of the HIV-1 antibody and HIV-2 antibody tests, the HTLV-1 antibody test, the HBs antigen test, and the HCV antibody test (excluding negative HCV-RNA) at the time of screening However, if the HCV antibody test is positive but HCV-RNA is negative, registration is allowed.

22. A patient who has a negative HBs antigen test but is positive for either an HBs antibody test or an HBc antibody test and has HBV-DNA quantification of not less than detection sensitivity, at the time of screening.

23. A patient who received the following treatments prior to giving consent and prior to administration of the investigational drug.

- A patient who has a history of treatment with pharmacotherapy including a drug targeting Magrolimab or the CD47-SIRPα pathway, Nivolumab, an anti-PD-1 antibody, an anti-PD-L1 antibody, an anti-PD-L2 antibody, an anti-CD137 antibody, an anti-CTLA-4 antibody, or other antibody therapy or cancer vaccine for the purpose of T cell control in the past.
- A patient who received more than 2 units of red blood cell transfusion within 28 days prior to consent acquisition.
- A patient who received surgical therapy with local anesthesia within 14 days prior to investigational drug administration.
- A patient who underwent adhesion surgery such as pleurodesis or pericardiodesis within 14 days prior to investigational drug administration.
- A patient who received administration of a >10 mg daily prednisolone-equivalent systemic adrenocortical hormone (except for temporary use for the purpose of examination, local administration, treatment and prevention of allergic reaction of contrast medium, or reduction of edema associated with radiotherapy, or the like) or immunosuppressive agent within 14 days prior to investigational drug administration.
- A patient who received radiation therapy for the purpose of palliation within 28 days prior to investigational drug administration.
- A patient who received surgical therapy with general anesthesia within 28 days prior to investigational drug administration.
- A patient who received live or attenuated vaccination within 28 days prior to investigational drug administration.
- A patient who received other unapproved drugs (including administration by clinical studies, unapproved combination drugs, and new dosage forms) within 28 days (or 90 days in a case of antibody formulation) prior to investigational drug administration.
- A patient who received a radiopharmaceutical (except for the use of a radiopharmaceutical for testing and diagnosis) within 56 days prior to investigational drug administration.

24. A patient known to have germline BRCA gene mutation (pathologic mutation or suspected pathologic mutation)

25. A patient who is pregnant, breastfeeding, or may be pregnant (a patient who is breastfeeding cannot be registered even if she stops breastfeeding).

26. A patient who is determined to be in a condition lacking consent ability due to dementia complication or the like.

27. In addition, a patient judged by the principal investigator or the co-investigator to be unsuitable as a research subject of the clinical trial.

(4) Dosage and duration of administration

[Magrolimab]

On Day 1 of the first cycle, 1 mg/kg of Magrolimab was intravenously administered over 3 hours ($\pm$ 30 minutes) as an initial administration, and then 20 mg/kg or 30 mg/kg of the same in a single does was intravenously administered over 2 hours ($\pm$ 30 minutes) at intervals of 1 week in the first cycle. In the second and subsequent cycles, 20 mg/kg or 30 mg/kg of Magrolimab in a single dose was intravenously administered over 2 hours ($\pm$ 30 minutes) at intervals of 2 weeks, and the administration was continued until predetermined administration stopping criteria for Magrolimab were met. In the case of administering Magrolimab, Nivolumab, and mFFX therapy on the same day, the administration was started in the order of Magrolimab, Nivolumab, and mFFX therapy.

[Nivolumab]

Nivolumab, 480 mg, was intravenously administered over 30 minutes at intervals of 4 weeks, and was continuously administered until predetermined administration stopping criteria for Nivolumab were met. The administration of Nivolumab was performed on and after Day 25, with an interval of at least 24 days from the previous administration.

[mFFX therapy]

(2a) 85 mg/m$^2$ (body surface area) of Oxaliplatin was intravenously administered over 2 hours. (2b) 200 mg/m$^2$ (body surface area) of Levofolinate calcium was intravenously administered over 2 hours. (2c) 150 mg/m$^2$ (body surface area) of Irinotecan hydrochloride hydrate was intravenously administered over 1.5 hours from 30 minutes after start of the administration of Levofolinate calcium. (2e) 2400 mg/m$^2$ (body surface area) of Fluorouracil was intravenously administered over 46 hours. The series of administrations in (2a), (2b), (2c), and (2e) above was performed at intervals of 2 weeks. Commercially available products were used for Oxaliplatin, Irinotecan hydrochloride hydrate, Levofolinate calcium, and Fluorouracil.

[Clinical trial schedule]

The present test consists of a screening period, a treatment period, and an observation period. The summary of the clinical trial schedule is shown in Fig. 2.

The screening period was within 28 days prior to investigational drug administration, and the principal investigator or co-investigator included, in the clinical trial, patients who met the above selection criteria, did not meet the above exclusion criteria, and were determined to be eligible for the clinical trial.

The treatment period was set to 28 days per cycle, and the first day of administration of the investigational drug was set to Day 1 of Cycle 1. The first day of each cycle after the second cycle is defined as Day [28 $\times$ (the number of cycles -1) + 1]. The administration of Magrolimab, Nivolumab, and mFFX therapy was started according to each dosage and administration, and the administration was continued according to respective administration criteria, dose-reduction criteria, and doses upon reduction for Magrolimab, Nivolumab, and mFFX therapy. The time point at which the evaluation at the completion (stop) of administration of Magrolimab, Nivolumab, and mFFX therapy was completed was defined as the end of the treatment period. Among all research subjects who received the investigational drug, research subjects to whom the administration of Magrolimab, Nivolumab, and the mFFX therapy was stopped or ended underwent evaluation at the end (stop) of administration and entered the follow-up period. Follow-up was performed after the completion of the observation period.

[Administration criteria for Magrolimab and Nivolumab]

At the start of each administration, research subjects must meet all predetermined administration criteria determined in view of the administration criteria in the clinical trials carried out to date regarding Magrolimab and Nivolumab. If any of these criteria were not met, the scheduled Magrolimab and Nivolumab administrations were withdrawn. The test was performed once a week or more frequently as clinically necessary for the research subject who had stopped taking the drug, to determine the possibility of restarting the administration.

[Administration stopping criteria for Magrolimab]

In the treatment period, the administration of Magrolimab was stopped for a research subject who met any of predetermined administration stopping criteria determined in view of the administration stopping criteria in the clinical trials carried out to date regarding Magrolimab.

[Administration stopping criteria for Nivolumab]

In the treatment period, the administration of Nivolumab was stopped for a research subject who met any of predetermined administration stopping criteria determined in view of the administration stopping criteria in the clinical trials carried out to date regarding Nivolumab.

[Administration Criteria of mFFX Therapy]

At the start of each administration, a research subject must meet all predetermined administration criteria determined in view of the latest package insert for mFFX therapy. The administration was postponed until the laboratory test values on the administration scheduled date recovered to states satisfying the conditions of the standard, and the administration was performed after confirming that it did not correspond to the contraindication of each drug.

[Dose reduction and dose reduction criteria for mFFX Therapy]

Dose reduction was performed according to the latest package insert.

[Stopping criteria for mFFX Therapy]

In the treatment period, for research subjects who met any of the predetermined administration stopping criteria determined in view of the latest package insert regarding mFFX therapy, the administration of mFFX therapy was stopped.

[Evaluation criteria of efficacy]

(Image diagnosis)

CT/nuclear magnetic resonance imaging (MRI) imaging and the like of the chest, abdomen and pelvis were performed.
The principal investigator or co-investigator measured the tumor diameter of the target lesion according to RECIST Guideline Version 1.1, and determined the antitumor effect.

(Evaluation items)

(1) Response rate (ORR), (2) disease control rate (DCR), (3) overall survival (OS), (4) progression-free survival (PFS), (5) duration of response (DOR), (6) time to response (TTR), (7) best overall response (BOR), (8) rate of change in sum of tumor diameters of target lesions, (9) maximum rate of change in sum of tumor diameters of target lesions, (10) changes in tumor markers (CEA and CA19 -9)

(Evaluation of target lesion)

Complete response (CR)

It means the disappearance of all non-lymph node target lesions and the reduction of the minor axis of all lymph node lesions selected as target lesions to less than 10 mm.

Partial response (PR)

This means that the sum of diameters of the target lesion decreases by 30% or more as compared with the baseline sum of diameters.

Progressive disease (PD)

This means a case where the sum of diameters of the target lesion is increased by 20% or more as compared with the minimum sum of diameters during the course, and the sum of diameters is increased by 5 mm or more as an absolute value.

Stable disease (SD)

This means a case where there is no reduction corresponding to PR and there is no increase corresponding to PD as compared with the minimum sum of diameters during the course.

Not Evaluable (NE)

This means a case where the test cannot be performed for some reason or a case where it cannot be determined as any of CR, PR, PD, and SD.

(1) Response rate

Response rate indicates the percentage of research subjects whose best overall response was determined to be CR or PR.

(2) Disease control rate

Disease control rate indicates the percentage of research subjects whose best overall response was determined to be CR, PR, or SD.

(3) Overall survival

The overall survival is calculated from the following equation.

$$\text{Overall survival (days)} = \text{"date of death due to any cause"} - \text{"date of starting administration of investigational drug"} + 1$$

Note that, for a research subject who became untraceable or who did not die by the data cut-off date, the final survival confirmation date is defined as the termination date.

(4) Progression-free survival

The progression-free survival is calculated from the following formula.

$$\text{Progression-free survival (days)} = \text{"earlier of date when overall effect was determined as PD and date of death due to any cause"} - \text{"date of starting administration of investigational drug"} + 1$$

Note that, for a research subject whose total effect has not been determined to be PD and who has not died, the date on which the last evaluable image diagnosis was performed is defined as the termination date. For a research subject who has not undergone evaluable diagnostic imaging and who has not died, the date of starting administration of investigational drug is defined as the termination date. For a research subject who received post-treatment for cancer before the overall effect was determined to be PD or died, the day on which the last evaluable imaging diagnosis before the start of post-treatment for cancer was performed is defined as the termination date.

(5) Duration of response

The duration of response is calculated from the following equation.

Duration of response (days) = "earlier of date when total response was determined to be PD for first time after confirmation of response, and date of death due to any cause" - "date of first determination of confirmed CR or PR" + 1

The subject to be evaluated is a research subject who shows CR or PR determined through the clinical trial.

(6) Time to response

The time to response is calculated from the following equation.

Time to response (days) = "date of first determination of confirmed CR or PR" - "date of starting administration of investigational drug" + 1

(7) Best overall response (BOR)

The best overall response is determined from the overall response determined by the completion of the clinical trial. However, CR and PR require confirmation by continuous evaluation after an interval of 4 weeks (28 days) or more, and are determined according to the criteria in Table 2.

[Table 2]

First overall evaluation
Subsequent overall evaluation
Best overall response
SD, PD, or PR
SD when satisfying minimum reference for SD, and PD in the other cases
SD when satisfying minimum reference for SD, and NE in the other cases

(8) Rate of change in sum of tumor diameters of target lesions

For a research subject having a target lesion, the rate of change in sum of tumor diameters of target lesions is calculated using the following calculation formula. However, the rate of change in sum of tumor diameters of target lesions after the post-treatment is performed is not calculated.

[Mathematical formula 4]

Rate of change (%) = ("sum of tumor diameters in each of evaluation time points – sum of tumor diameters before administration") / ("sum of tumor diameters before administration") x 100

(9) Maximum rate of change in sum of tumor diameters of target lesions

The maximum rate of change in sum of tumor diameters of target lesions is defined as a rate of change at the time point when the sum of tumor diameters of the target lesions is the smallest. However, the sum of the tumor diameters of the target lesions after the total effect is determined to be PD or after the post-treatment is performed is not used for calculating the maximum rate of change.

[Mathematical formula 5]

$$\text{Maximum rate of change (\%)} = (\text{``smallest sum of tumor diameters after administration - sum of tumor diameters before administration''}) / (\text{``sum of tumor diameters before administration''}) \times 100$$

(10) Change in rate of change in tumor marker (CEA and CA19-9)

The rate of change in the tumor marker is calculated using the following calculation formula. However, the rate of change in the tumor marker after the post-treatment is performed is not calculated.

[Mathematical formula 6]

$$\text{Rate of change (\%)} = (\text{``tumor marker in each of evaluation time points - tumor marker before administration''}) / (\text{``tumor marker before administration''}) \times 100$$

[Evaluation items of safety]

The following items were measured, examined and investigated by the principal investigator and the like at a predetermined time.
(1) Dose-limiting toxicity (DLT), (2) adverse events, (3) laboratory tests (hematological tests, blood biochemical tests, immunological tests, hormonal tests, blood coagulation system tests, urine qualitative tests), peripheral blood smear, (4) vital signs (systolic/diastolic blood pressure, pulse rate, body temperature, respiratory rate), transcutaneous oxygen saturation ($SpO_2$), body weight, (5) 12-lead electrocardiogram, (6) ECOG Performance Status, and (7) chest x-ray

[Results of efficacy evaluation]

For Magrolimab, Nivolumab, and a combination therapy which is mFFX therapy, there was one case determined to be PR at the time point of 10 months after the registration of the first research subject.

INDUSTRIAL APPLICABILITY

The present invention provides a new cancer treatment method and is useful.

**Claims**

1. An agent for suppressing progression of, suppressing recurrence of, and/or treating a solid cancer, comprising a CD47 inhibitory substance as an active ingredient, wherein the agent is administered in combination with a standard therapy and an immune checkpoint inhibitory substance.

2. An agent for suppressing progression of, suppressing recurrence of, and/or treating a solid cancer, comprising an immune checkpoint inhibitory substance as an active ingredient, wherein the agent is administered in combination with a standard therapy and a CD47 inhibitory substance.

3. The agent according to claim 1 or 2, wherein the standard therapy is a combination therapy in which Bevacizumab or Cetuximab is added to FOLFOX therapy.

4. The agent according to claim 3, wherein
the combination therapy in which Bevacizumab or Cetuximab is added to FOLFOX therapy includes:

(1x) intravenously administering 5 mg/kg (body weight) of Bevacizumab, or intravenously administering 400 mg/m² (body surface area) of Cetuximab for a first administration, and 250 mg/m² (body surface area) of Cetuximab for a second and subsequent administrations;
(1a) intravenously administering 85 mg/m² (body surface area) of Oxaliplatin;

(1b) intravenously administering 200 mg/m$^2$ (body surface area) of Levofolinate calcium;
(1c) rapidly intravenously administering 400 mg/m$^2$ (body surface area) of Fluorouracil; and
(1d) further continuously intravenously administering 2400 mg/m$^2$ (body surface area) of Fluorouracil after completion of the administration of Fluorouracil in (1c) above, and
a series of administrations in (1x), (1a), (1b), (1c), and (1d) above is performed at intervals of 2 weeks.

5.  The agent according to claim 4, wherein
the combination therapy in which Bevacizumab or Cetuximab is added to FOLFOX therapy includes:

(1x) intravenously administering 5 mg/kg (body weight) of Bevacizumab;
(1a) intravenously administering 85 mg/m$^2$ (body surface area) of Oxaliplatin;
(1b) intravenously administering 200 mg/m$^2$ (body surface area) of Levofolinate calcium;
(1c) rapidly intravenously administering 400 mg/m$^2$ (body surface area) of Fluorouracil; and
(1d) further continuously intravenously administering 2400 mg/m$^2$ (body surface area) of Fluorouracil after completion of the administration of Fluorouracil in (1c) above, and
a series of administrations in (1x), (1a), (1b), (1c), and (1d) above is performed at intervals of 2 weeks.

6.  The agent according to claim 4, wherein

the combination therapy in which Bevacizumab or Cetuximab is added to FOLFOX therapy includes:

(1x) intravenously administering 400 mg/m$^2$ (body surface area) of Cetuximab for a first administration, and 250 mg/m$^2$ (body surface area) of Cetuximab for a second and subsequent administrations;
(1a) intravenously administering 85 mg/m$^2$ (body surface area) of Oxaliplatin;
(1b) intravenously administering 200 mg/m$^2$ (body surface area) of Levofolinate calcium;
(1c) rapidly intravenously administering 400 mg/m$^2$ (body surface area) of Fluorouracil; and
(1d) further continuously intravenously administering 2400 mg/m$^2$ (body surface area) of Fluorouracil after completion of the administration of Fluorouracil in (1c) above, and

a series of administrations in (1x), (1a), (1b), (1c), and (1d) above is performed at intervals of 2 weeks.

7.  The agent according to any one of claims 1 to 6, wherein the solid cancer is colorectal cancer.

8.  The agent according to claim 7, wherein the colorectal cancer is radically unresectable advanced or recurrent colorectal cancer.

9.  The agent according to any one of claims 1 to 8, wherein the agent is administered as a primary therapy to a patient who has not been treated with a systemic anti-malignant tumor agent against radically unresectable advanced or recurrent colorectal cancer.

10. The agent according to claim 1 or 2, wherein the standard therapy is FOLFIRINOX therapy or its dose-reduced regimen.

11. The agent according to claim 10, wherein

FOLFIRINOX therapy includes:

(2a) intravenously administering 85 mg/m$^2$ (body surface area) of Oxaliplatin;
(2b) intravenously administering 200 mg/m$^2$ (body surface area) of Levofolinate calcium;
(2c) intravenously administering 180 mg/m$^2$ (body surface area) of Irinotecan hydrochloride hydrate;
(2d) rapidly intravenously administering 400 mg/m$^2$ (body surface area) of Fluorouracil; and
(2e) further continuously intravenously administering 2400 mg/m$^2$ (body surface area) of Fluorouracil after completion of the administration of Fluorouracil in (2d) above, and

a series of administrations in (2a), (2b), (2c), (2d), and (2e) above is performed at intervals of 2 weeks.

12. The agent according to claim 10, wherein

the dose-reduced regimen of FOLFIRINOX therapy includes:

(2a) intravenously administering 85 mg/m$^2$ (body surface area) of Oxaliplatin;
(2b) intravenously administering 200 mg/m$^2$ (body surface area) of Levofolinate calcium;
(2c) intravenously administering 150 mg/m$^2$ (body surface area) of Irinotecan hydrochloride hydrate; and
(2e) continuously intravenously administering 2400 mg/m$^2$ (body surface area) of Fluorouracil, and

a series of administrations in (2a), (2b), (2c), and (2e) above is performed at intervals of 2 weeks.

13. The agent according to any one of claims 1, 2, 10, 11, and 12, wherein the solid cancer is pancreatic cancer having distant metastasis.

14. The agent according to any one of claims 1, 2, 10, 11, 12, and 13, wherein the agent is administered as a primary therapy to a patient who has not been treated with a systemic anti-malignant tumor agent against pancreatic cancer having distant metastasis.

15. The agent according to any one of claims 1 to 14, wherein the CD47 inhibitory substance is an anti-CD47 antibody.

16. The agent according to claim 15, wherein the anti-CD47 antibody is Magrolimab.

17. The agent according to claim 16, wherein 1 mg/kg (body weight) of Magrolimab is intravenously administered for a first administration, and 15 mg/kg (body weight) to 30 mg/kg (body weight) of Magrolimab in a single dose is intravenously administered at intervals of 1 week or 2 weeks for a second and subsequent administrations.

18. The agent according to claim 16 or 17, wherein 1 mg/kg (body weight) of Magrolimab is intravenously administered in a first administration, and 30 mg/kg (body weight) of Magrolimab in a single dose is intravenously administered at intervals of 1 week or 2 weeks in a second and subsequent administrations.

19. The agent according to any one of claims 1 to 18, wherein the immune checkpoint inhibitory substance is an anti-PD-1 antibody, an anti-PD-L1 antibody, or an anti-CTLA-4 antibody.

20. The agent according to any one of claims 1 to 19, wherein the immune checkpoint inhibitory substance is an anti-PD-1 antibody.

21. The agent according to claim 19 or 20, wherein the anti-PD-1 antibody is Nivolumab, Cemiplimab, Pembrolizumab, Spartalizumab, Tislelizumab, AMP-514, Dostartimab, Toripalimab, Camrelizumab, Genolimzumab, Sintilimab, STI-A1110, ENUM 388D4, ENUM 244C8, GLS010, Retifanlimab, Balstilimab, CS1003, Serplulimab, BAT-1306, AK105, AK103, BI 754091, LZM009, CMAB819, Sym021, Geptanolimab, SSI-361, JY034, HX008, ISU106, Budigalimab, Prolgolimab, Sasanlimab, CX-188, Cetrelimab, or Zimberelimab.

22. The agent according to claim 21, wherein the anti-PD-1 antibody is Nivolumab.

23. The agent according to claim 22, wherein 480 mg of Nivolumab in a single dose is intravenously administered at intervals of 4 weeks.

*FIG. 1*

**Screening Period**

colorectal cancer

No chemotherapy treatment
or 180 days or later
after completion of adjuvant
therapy
Microsatellites Stable
Proficient mismatch repair
BRAF wild type

**Treatment Period**

**Cohort 1 (RAS mutant)**
Magrolimab (Treatment Level A or B)
Nivolumab 480 mg every 4 weeks
FOLFOX
Bevacizumab

**Cohort 2 (RAS wild type)**
Magrolimab (Treatment Level A or B)
Nivolumab 480 mg every 4 weeks
FOLFOX
Cetuximab

Treatment
for up to
24 months
until
stopping
criteria are
met

**Follow-up Period**

Follow-up

**Tolerability assessment part**

**Treatment Level A**
Magrolimab (Treatment Level A or B)
Nivolumab 480 mg every 4 weeks
FOLFOX
Bevacizumab (Cohort 1)
Cetuximab (Cohort 2)

Start at Treatment Level A and, if not
tolerated, proceed to Treatment Level B

**Treatment Level B**
Magrolimab (Treatment Level A or B)
Nivolumab 480 mg every 4 weeks
FOLFOX
Bevacizumab (Cohort 1)
Cetuximab (Cohort 2)

FOLFOX : Fluorouracil, Levofolinate, Oxaliplatin

**Expansion part**

**Treatment Level A or B**
Magrolimab (Treatment Level A or B)
Nivolumab 480 mg every 4 weeks
FOLFOX
Bevacizumab (Cohort 1)
Cetuximab (Cohort 2)

EP 4 353 265 A1

FIG.2

【Tolerability assessment part】 　　　　　【Expansion part】

【Cohort 1】
Magrolimab
　1 mg/kg （primary Dose）
→30 mg/kg （Maintenance Dose）
Nivolumab 480 mg
mFFX therapy

The study starts at a maintenance dose of 30 mg/kg
If the study is not tolerated
Moved to a maintenance dose of 20 mg/kg of Magrolimab

【Cohort 2】
Magrolimab
　1 mg/kg （primary Dose）
→30 mg/kg （Maintenance Dose）
Nivolumab 480 mg
mFFX therapy

Magrolimab
　　　　Maintenance Dose
Nivolumab 480 mg
mFFX thrapy

screening period | treatment period*1 | follow-up period

←— 1 cycle —→ ←— 2 cycle —→ ←— 3 cycle~ —→ stop

Day　1　8　15　22　1　8　15　22　1　8　15　22　. . .

follow-up

Magrolimab*2

Nivolumab*3

mFFX*4

Treatment may be repeated up to 24 months until treatment discontinuation criteria are met.

*1: 1 cycle (4 weeks)
*2: Magrolimab administered weekly in Cycle 1 and every 2 weeks thereafter
*3: If administered on the same day as Magrolimab, start Magrolimab first
*4: If administered on the same day as Magrolimab and/or Nivolumab, then finally start mFFX therapy.

## INTERNATIONAL SEARCH REPORT

| International application No. |
|---|
| **PCT/JP2022/023300** |

### A. CLASSIFICATION OF SUBJECT MATTER

*A61K 45/06*(2006.01)i; *A61K 31/282*(2006.01)i; *A61K 31/4745*(2006.01)i; *A61K 31/513*(2006.01)i; *A61K 31/519*(2006.01)i; *A61K 39/395*(2006.01)i; *A61P 1/04*(2006.01)i; *A61P 1/18*(2006.01)i; *A61P 35/00*(2006.01)i; *A61P 35/04*(2006.01)i; *A61P 37/02*(2006.01)i; *A61P 43/00*(2006.01)i
FI: A61K45/06; A61K31/282; A61K31/4745; A61K31/513; A61K31/519; A61K39/395 N; A61P1/04; A61P1/18; A61P35/00; A61P35/04; A61P37/02; A61P43/00 111; A61P43/00 121

According to International Patent Classification (IPC) or to both national classification and IPC

### B. FIELDS SEARCHED

Minimum documentation searched (classification system followed by classification symbols)

A61K45/06; A61K31/282; A61K31/4745; A61K31/513; A61K31/519; A61K39/395; A61P1/04; A61P1/18; A61P35/00; A61P35/04; A61P37/02; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | JP 2015-519372 A (GENENTECH, INC.) 09 July 2015 (2015-07-09) claims, examples, paragraphs [0084], [0098] | 1-9, 15-23 |
| A | | 10-14 |
| Y | WO 2020/111018 A1 (ONO PHARMACEUTICAL CO., LTD.) 04 June 2020 (2020-06-04) claims | 1, 2, 10-23 |
| A | | 3-9 |
| Y | KUO, KUO, T. C. et al. Targeting the myeloid checkpoint receptor SIRPα potentiates innate and adaptive immune responses to promote anti-tumor activity. J. Hematol. Oncol. 2020, vol. 13, no. 1, article.160<DOI:10.1186/s13045-020-00989-w> abstract, page 11, left column, second paragraph | 1-23 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **05 August 2022** | **23 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/023300**

| Patent document cited in search report | | | Publication date (day/month/year) | Patent family member(s) | | | Publication date (day/month/year) |
|---|---|---|---|---|---|---|---|
| JP | 2015-519372 | A | 09 July 2015 | US | 2015/0320859 | A1 | |
| | | | | claims, examples, paragraphs [0087], [0101] | | | |
| | | | | WO | 2013/181452 | A1 | |
| | | | | EP | 2855528 | A1 | |
| | | | | CN | 104271601 | A | |
| | | | | KR | 10-2015-0027135 | A | |
| WO | 2020/111018 | A1 | 04 June 2020 | US | 2022/0008421 | A1 | |
| | | | | claims | | | |
| | | | | EP | 3888648 | A1 | |
| | | | | KR | 10-2021-0095868 | A | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009091601 A
- WO 2011143624 A
- WO 2006121168 A
- WO 2008156712 A
- WO 2007005874 A
- WO 2001014424 A

**Non-patent literature cited in the description**

- *Trends in Cell Biology,* 2001, vol. 11 (3), 130-135
- *Journal of Experimental Medicine,* 2001, vol. 194 (4), 541-549
- *Journal of Immunology,* 2005, vol. 174 (4), 2004-2011
- *Cell,* 2009, vol. 138 (2), 286-299
- *CHEMICAL ABSTRACTS,* 2169232-81-7
- *Nature Reviews Cancer,* 2012, vol. 12, 252-264
- *Cancer Cell,* 2015, vol. 27, 450-461